# EUROPEAN PATENT APPLICATION

(11) **EP 1 887 082 A2**
(43) Date of publication of application: **13.02.2008**
(21) Application number: 07118615.9
(22) Date of filing: 20.10.2000
(51) Int. Cl.: C12N 15/55, C12Q 1/68, C12P 21/06, C12N 15/00, C12N 15/09

(54) **TADG-15: An extracellular serine protease overexpressed in carcinomas**

(30) Priority: 20.10.1999 US 421213
(62) Divisional of application: 00973739.6
(71) Applicant: THE BOARD OF TRUSTEES OF THE UNIVERSITY OF ARKANSAS, Little Rock, AR 72207-3608 (US)
(72) Inventor: O'Brien, Timothy J., Little Rock, AR 72207 (US); Tanimoto, Hirotoshi, Marugame, Kagawa 763-0033 (JP)
(74) Representative: McQueen, Andrew Peter

(57) **Abstract**

The present invention provides DNA encoding a TADG-15 protein as well as a TADG-15 protein. Also provided is a vector capable of expressing the DNA of the present invention adapted for expression in a recombinant cell and regulatory elements necessary for expression of the DNA in the cell. The present invention further provides for methods of inhibiting TADG-15 expression and/or protease activity, methods of detecting TADG-15 mRNA and/or protein and methods of screening for TADG-15 inhibitors. Additionally, the present invention provides for cell-specific targeting via TADG-15 and methods of vaccinating an individual against TADG-15. The methods described are useful in the diagnosis, treatment and prevention of cancer, particularly breast and ovarian cancer.

## Description

### BACKGROUND OF THE INVENTION

### Cross-Reference to Related Application

This application in a continuation-in-part of USSN 09/027,337, filed February 20, 1998 and thereby claims the benefit of priority under 35 USC §120.

### Field of the Invention

The present invention relates generally to the fields of cellular biology and the diagnosis of neoplastic disease. More specifically, the present invention relates to an extracellular serine protease, termed tumor antigen-derived gene 15 (TADG-15), which is overexpressed in carcinomas.

### Description of the Related Art

Extracellular proteases have been directly associated with tumor growth, shedding of tumor cells and invasion of target organs. Individual classes of proteases are involved in, but not limited to, (a) digestion of stroma surrounding the initial tumor area, (b) digestion of the cellular adhesion molecules to allow dissociation of tumor cells; and (c) invasion of the basement membrane for metastatic growth and activation of both tumor growth factors and angiogenic factors.

In the process of cancer progression and invasion, proteases mediate specific proteolysis and contribute to the removal of extracellular matrix components surrounding tumor cells, the digestion of intercellular adhesion molecules to allow dissociation of malignant cells and the activation of many growth and angiogenic factors.¹⁻³ Depending on the nature of their catalytic domain, proteases are classified into four families: serine proteases, metalloproteases, aspartic proteases and cysteine proteases.³ Among these proteases, the metalloproteases have been well studied in relation to tumor growth and progression, and they are known to be capable of degrading the extracellular matrix, thereby enhancing the invasive potential of malignant cells.^{1,4,5} For serine proteases, previous studies have demonstrated an increased production of plasminogen activator in tumor cells and a positive correlation between plasminogen activator activity and aggressiveness of cancer. ^{6,7} Prostate specific antigen (a serine protease) has also been widely used as an indicator of abnormal prostate growth.⁸ More recently, several other serine proteases have been reported, *viz.* hepsin and the stratum corneum chymotryptic enzyme (SCCE), which are overexpressed in ovarian cancer and which may contribute to malignant progression by increasing the extracellular lytic activity of these tumor cells.⁹

The prior art is deficient in the lack of effective means of screening to identify proteases overexpressed in carcinoma. The present invention fulfills this longstanding need and desire in the art.

### SUMMARY OF THE INVENTION

The present invention discloses a screening program to identify proteases overexpressed in carcinoma by examining PCR products amplified using differential display in early stage tumors and metastatic tumors compared to that of normal tissues. The approach herein to identify candidate genes overexpressed in tumor cells has been to utilize the well conserved domains surrounding the triad of amino acids (His-Asp-Ser) prototypical of the catalytic domain of serine proteases. Herein, evidence is presented for a unique form of serine protease not previously described in the literature which is highly expressed in ovarian carcinomas. Through the screening approach using differential PCR amplification of normal, low malignant potential and overt carcinomas, a PCR product present only in carcinoma was subcloned and sequenced, and was found to have a catalytic domain which was consistent with the serine protease family. Reported herein is the complete cloning and sequencing of this transcript and evidence for its expression in ovarian tumor cells.

In one embodiment of the present invention, there is provided a DNA encoding a tumor antigen-derived gene (TADG-15) protein, selected from the following: (a) an isolated DNA which encodes a TADG-15 protein; (b) an isolated DNA which hybridizes under high stringency conditions to the isolated DNA of (a) above and which encodes a TADG-15 protein; and (c) an isolated DNA differing from the isolated DNAs of (a) and (b) above in codon sequence due to the degeneracy of the genetic code, and which encodes a TADG-15 protein. The embodiment further includes a vector comprising the TADG-15 DNA and regulatory elements necessary for expression of the DNA in a cell. Additionally embodied is a vector in which the TADG-15 DNA is positioned in reverse orientation relative to the regulatory elements such that TADG-15 antisense mRNA is produced.

In another embodiment of the present invention, there is provided an isolated and purified TADG-15 protein coded for by DNA selected from the following: (a) an isolated DNA which encodes a TADG-15 protein; (b) an isolated DNA which hybridizes under high stringency conditions to isolated DNA of (a) above and which encodes a TADG-15 protein; and (c) an isolated DNA differing from the isolated DNAs of (a) and (b) above in codon sequence due to the degeneracy of the genetic code, and which encodes a TADG-15 protein.

In yet another embodiment of the present invention, there is provided a method for detecting TADG-15 mRNA in a sample, comprising the steps of: (a) contacting a sample with a probe which is specific for TADG-15; and (b) detecting binding of the probe to TADG-15 mRNA in the sample. In still yet another embodiment of the present invention, there is provided a kit for detecting TADG-15 mRNA, comprising: an oligonucleotide probe specific for TADG-15. A label for detection is further embodied in the kit.

The present invention additionally embodies a method of detecting TADG-15 protein in a sample, comprising the steps of: (a) contacting a sample with an antibody which is specific for TADG-15 or a fragment thereof; and (b) detecting binding of the antibody to TADG-15 protein in the sample. Similarly, the present invention also embodies a kit for detecting TADG-15 protein, comprising: an antibody specific for TADG-15 protein or a fragment thereof. Means for detection of the antibody is further embodied in the kit.

In another embodiment, the present invention provides an antibody specific for the TADG-15 protein or a fragment thereof.

In yet another embodiment, the present invention provides a method of screening for compounds that inhibit TADG-15, comprising the steps of: (a) contacting a sample comprising TADG-15 protein with a compound; and (b) assaying for TADG-15 protease activity. Typically, a decrease in the TADG-15 protease activity in the presence of the compound relative to TADG-15 protease activity in the absence of the compound is indicative of a compound that inhibits TADG-15.

In still yet another embodiment of the present invention, there is provided a method of inhibiting expression of TADG-15 in a cell, comprising the step of: (a) introducing a vector into a cell, whereupon expression of the vector produces TADG-15 antisense mRNA in the cell which hybridizes to endogenous TADG-15 mRNA, thereby inhibiting expression of TADG-15 in the cell.

Further embodied by the present invention, there is provided a method of inhibiting a TADG-15 protein in a cell, comprising the step of: (a) introducing an antibody specific for a TADG-15 protein or a fragment thereof into a cell, whereupon binding of the antibody to the TADG-15 protein inhibits the TADG-15 protein.

In an embodiment of the present invention, there is provided a method of targeted therapy to an individual, comprising the step of: (a) administering a compound containing a targeting moiety and a therapeutic moiety to an individual, wherein the targeting moiety is specific for TADG-15.

In an embodiment of the present invention, there is provided a method of diagnosing cancer in an individual, comprising the steps of: (a) obtaining a biological sample from an individual; and (b) detecting TADG-15 in the sample, wherein the presence of TADG-15 in the sample is indicative of the presence of carcinoma in the individual and the absence of TADG-15 in the sample is indicative of the absence of carcinoma in the individual.

In another embodiment of the present invention, there is provided a method of vaccinating an individual against TADG-15, comprising the steps of: (a) inoculating an individual with a TADG-15 protein or fragment thereof that lacks TADG-15 protease activity, wherein the inoculation with the TADG-15 protein or fragment thereof elicits an immune response in the individual, thereby vaccinating the individual against TADG-15.

In an embodiment of the present invention, there is provided a method of producing immune-activated cells directed toward TADG-15, comprising the steps of: exposing dendritic cells to a TADG-15 protein or fragment thereof that lacks TADG-15 protease activity, wherein the exposure to said TADG-15 protein or fragment thereof activates the dendritic cells, thereby producing immune-activated cells directed toward TADG-15.

In another embodiment of the present invention, there is provided an immunogenic composition, comprising an immunogenic fragment of a TADG-15 protein and an appropriate adjuvant.

Other and further aspects, features, and advantages of the present invention will be apparent from the following description of the presently preferred embodiments of the invention given for the purpose of disclosure.

### BRIEF DESCRIPTION OF THE DRAWINGS

So that the matter in which the above-recited features, advantages and objects of the invention, as well as others which will become clear, are attained and can be understood in detail, more particular descriptions of the invention briefly summarized above may be had by reference to certain embodiments thereof which are illustrated in the appended drawings. These drawings form a part of the specification. It is to be noted, however, that the appended drawings illustrate preferred embodiments of the invention and therefore are not to be considered limiting in their scope.

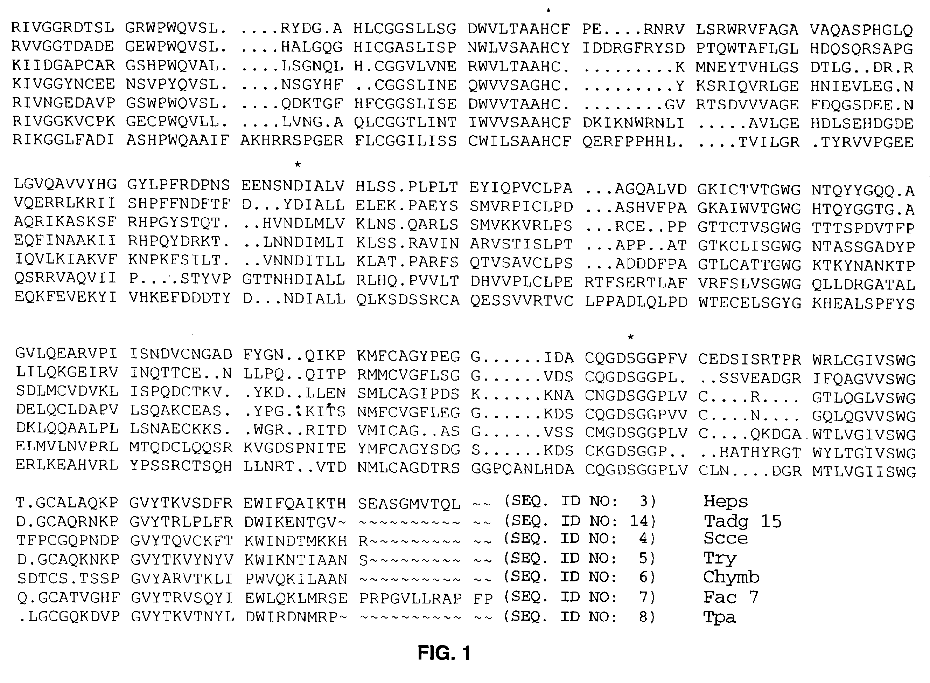

**Figure 1** shows a comparison of the serine protease catalytic domain of TADG-15 with Hepsin (Heps, SEQ ID No. 3), SCCE (SEQ ID No. 4), Trypsin (Try, SEQ ID No. 5), Chymotrypsin (Chymb, SEQ ID No. 6), Factor 7 (Fac7, SEQ ID No. 7) and Tissue plasminogen activator (Tpa, SEQ ID No. 8). The asterisks indicate conserved amino acids of catalytic triad.

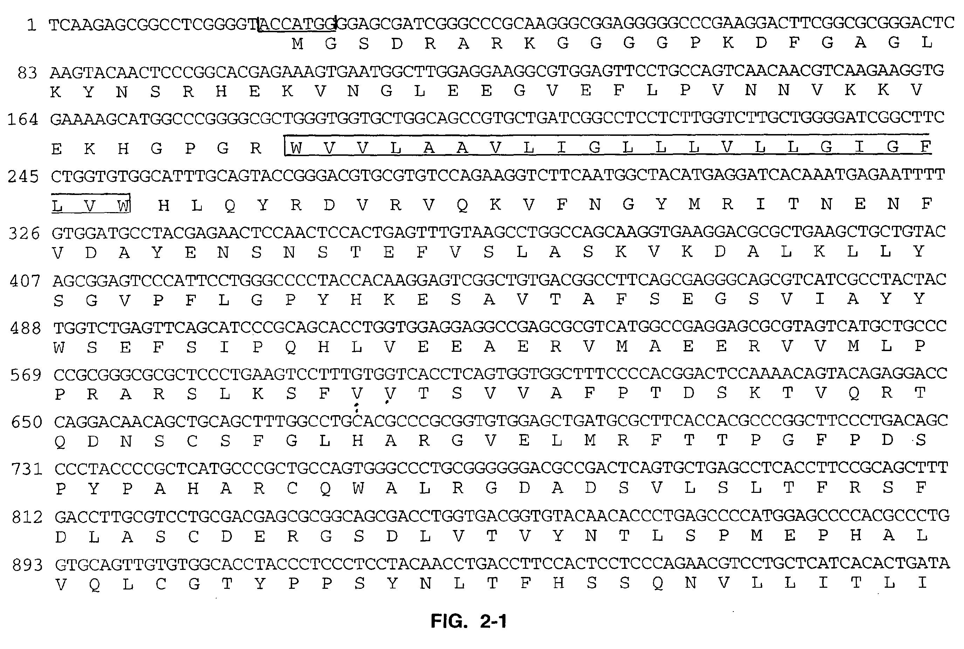

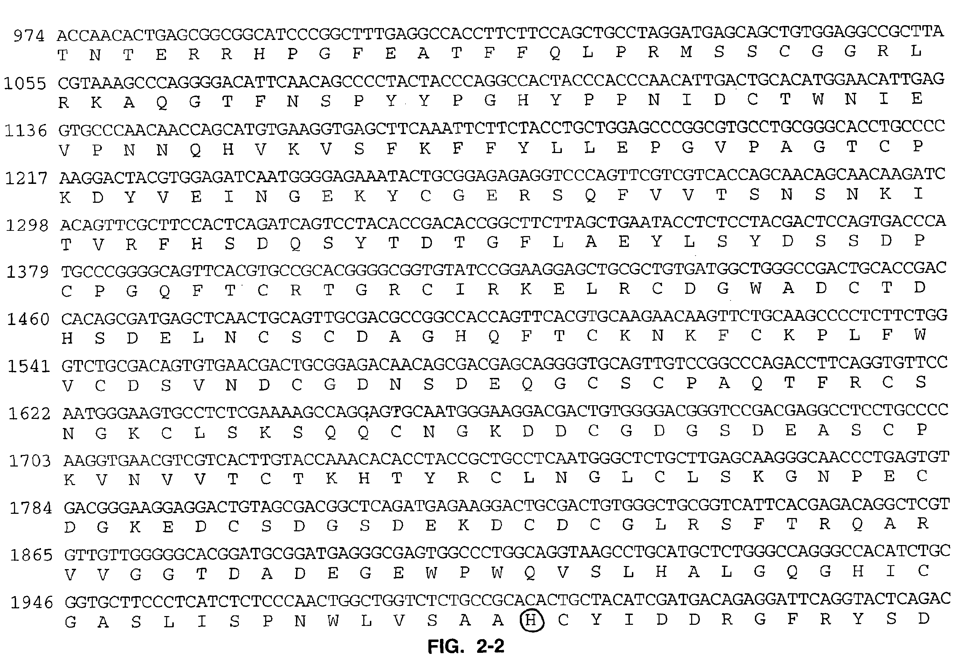

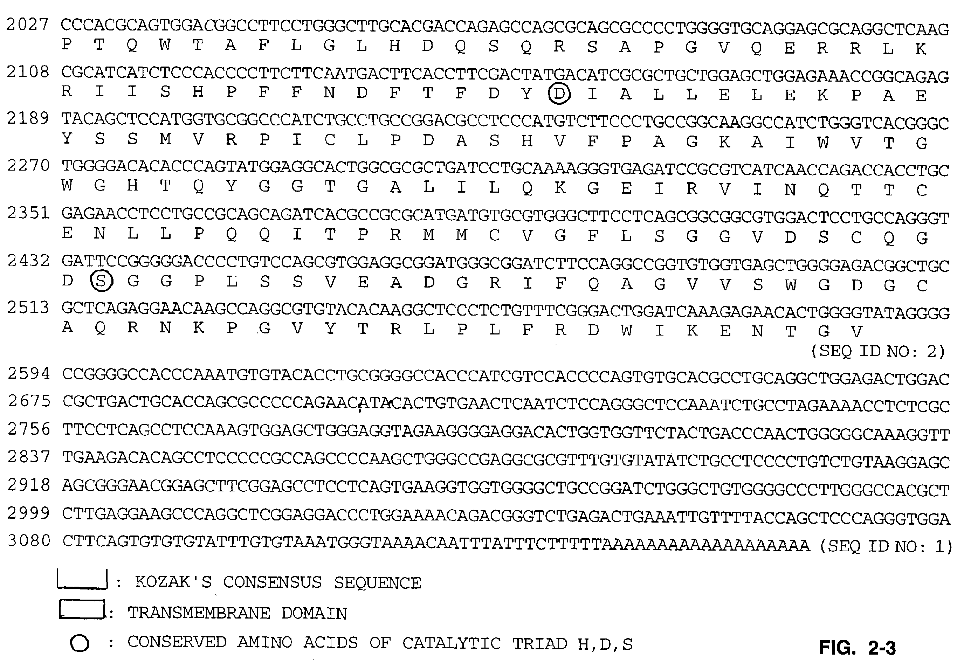

**Figure 2** shows the nucleotide sequence of the TADG-15 cDNA and the derived amino acid sequence of the TADG-15 protein. The putative start codon is located at nucleotides 23-25. The potential transmembrane sequence is underlined. Possible N-linked glycosylation sites are indicated by a broken line. The asterisks indicate conserved cysteine residues of CUB domain. The SDE-motifs of the LDL receptor ligand binding repeat-like domain are boxed. The arrow shows the arginine-valine bond cleaved upon activation. The conserved amino acids of the catalytic triad; histidine, aspartic acid and serine residues are circled.

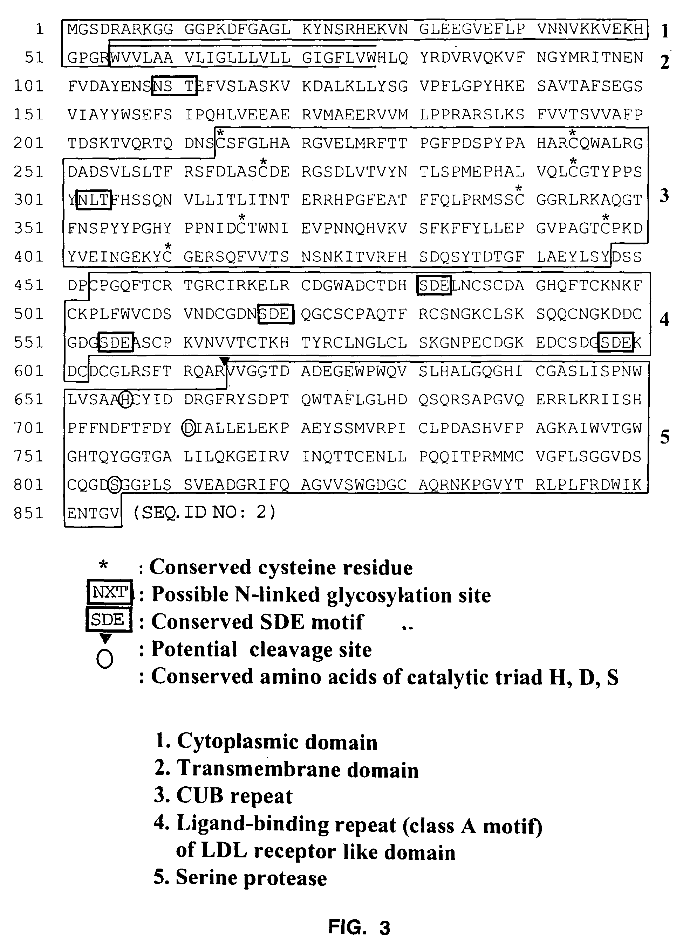

**Figure 3** shows the amino acid sequence of the TADG-15 protease, including functional sites and domains.

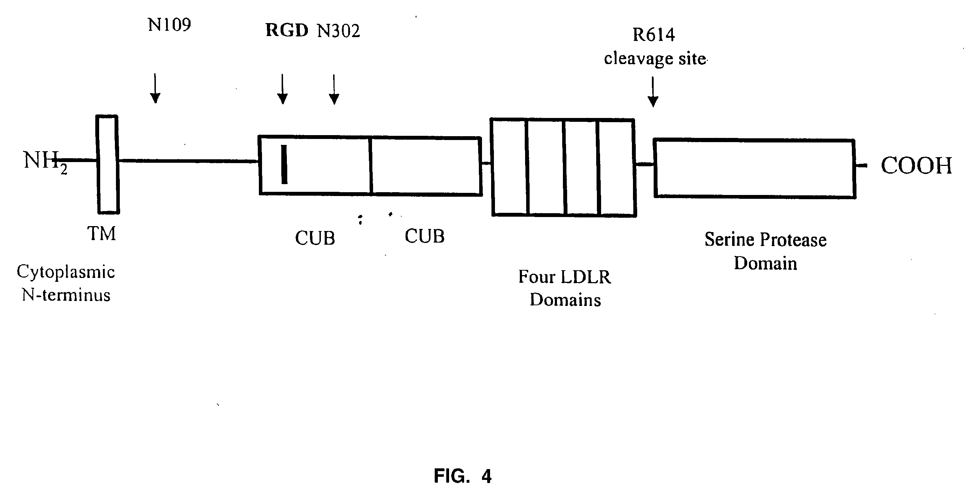

**Figure 4** shows a diagram of the TADG-15 protein. 1; cytoplasmic domain, (aa #1-54), 2; transmembrane domain (aa #55-57), 3; extracellular domain (aa #78-213), 4-5; CUB repeat (aa #214-447), 6-9; LDL receptor ligand binding repeat (class A motif) like domain (aa #453-602), 10; serine protease (aa #615-855).

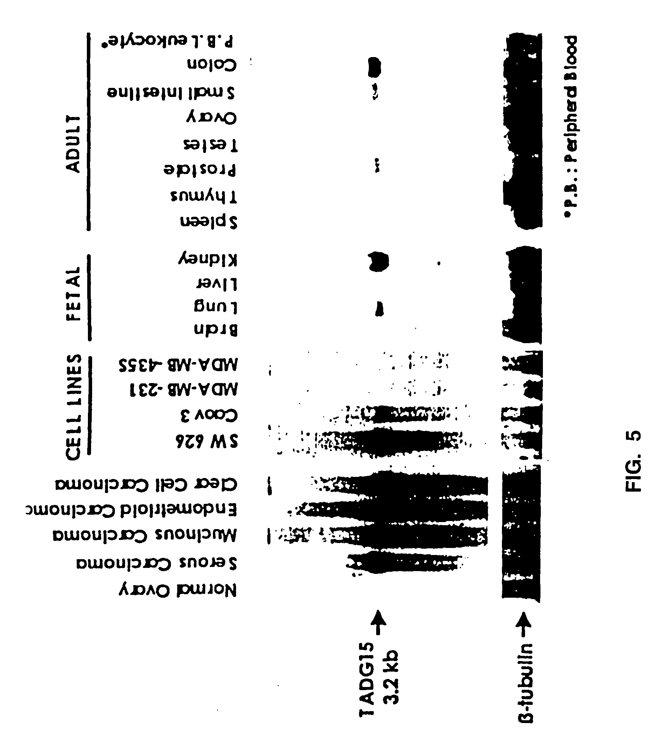

**Figure 5** shows Northern blot analysis of TADG-15 mRNA expression in normal ovary, ovarian carcinomas, carcinoma cell lines, normal fetal tissues and normal adult tissues. A single intense transcript of the TADG-15 was observed in every sub-type of carcinoma and the two ovarian carcinoma cell lines, SW626 and CAOV3, whereas no visible band was detected in normal ovary or the two breast cancer cell lines. In normal fetal tissues, fetal kidney showed increased transcript and faint expression was detected in fetal lung. In normal adult tissues, the TADG-15 was detected in colon with low expression in small intestine and prostate.

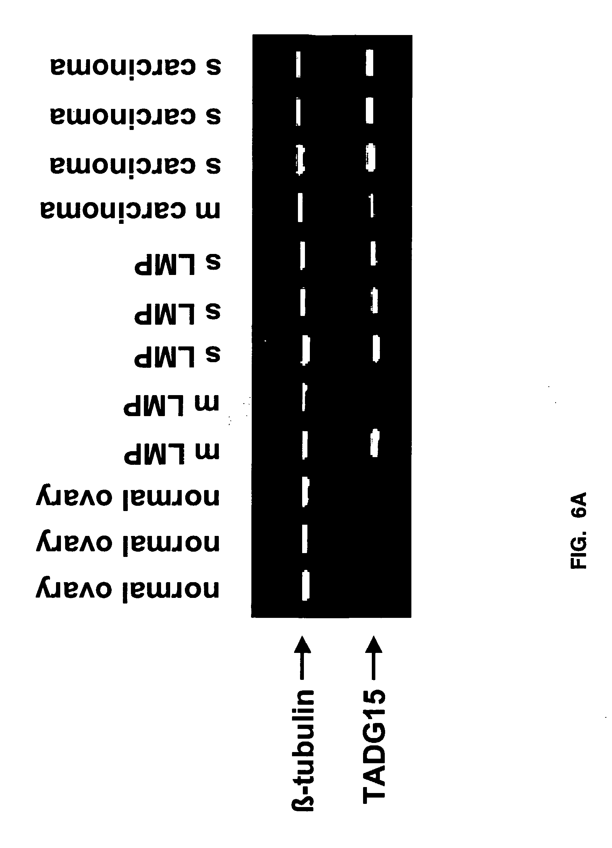

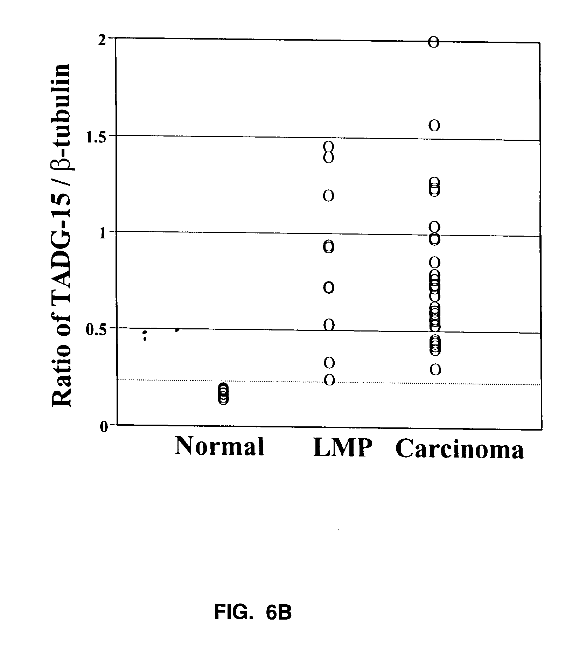

**Figure 6A** shows quantitative PCR analysis of TADG-15 expression. Expression levels of TADG-15 relative to β-tubulin are significantly elevated in all LMP tumors and carcinomas compared to that of normal ovaries. m; mucinous, s; serous. **Figure 6B** shows the ratio of TADG-15 expression to expression of β-tubulin in normal ovary, LMP tumor and ovarian carcinoma. TADG-15 mRNA expression levels were significantly elevated in both LMP tumor (*; p<0.001) and carcinoma (**; p<0.0001) compared to that in normal ovary. All 10 samples of normal ovary showed a low level of TADG-15 expression.

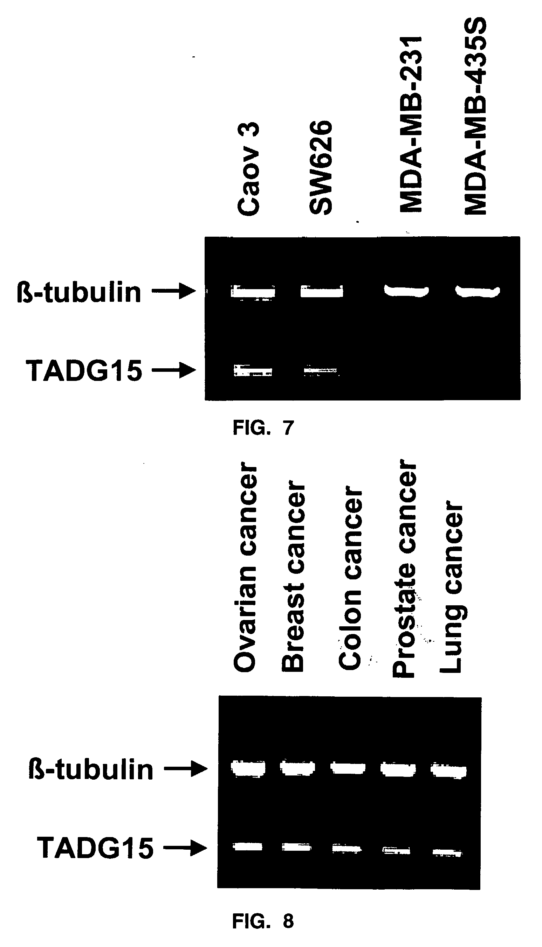

**Figure 7** shows the TADG-15 expression in tumor cell lines derived from both ovarian and breast carcinoma tissues.
**Figure 8** shows the overexpression of TADG-15 in other tumor tissues.

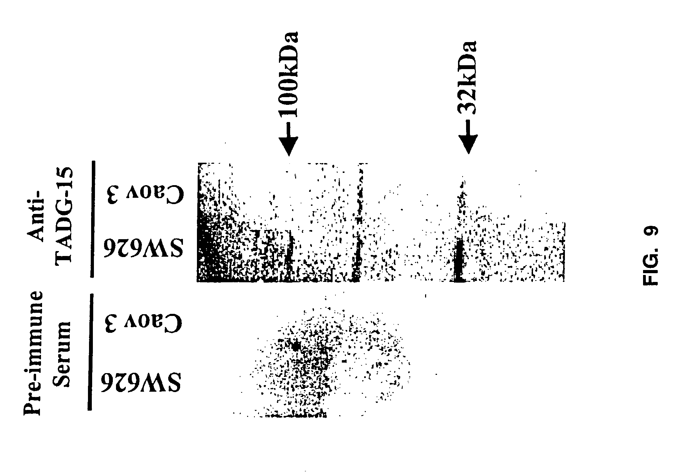

**Figure 9** shows SW626 and CAOV3 cell lysates that were separated by SDS-PAGE and immunoblotted. Lanes 1 and 2 were probed with rabbit pre-immune serum as a negative control. Lanes 3 and 4 were probed with polyclonal rabbit antibody generated to a carboxy terminal peptide from TADG-15 protein sequence.

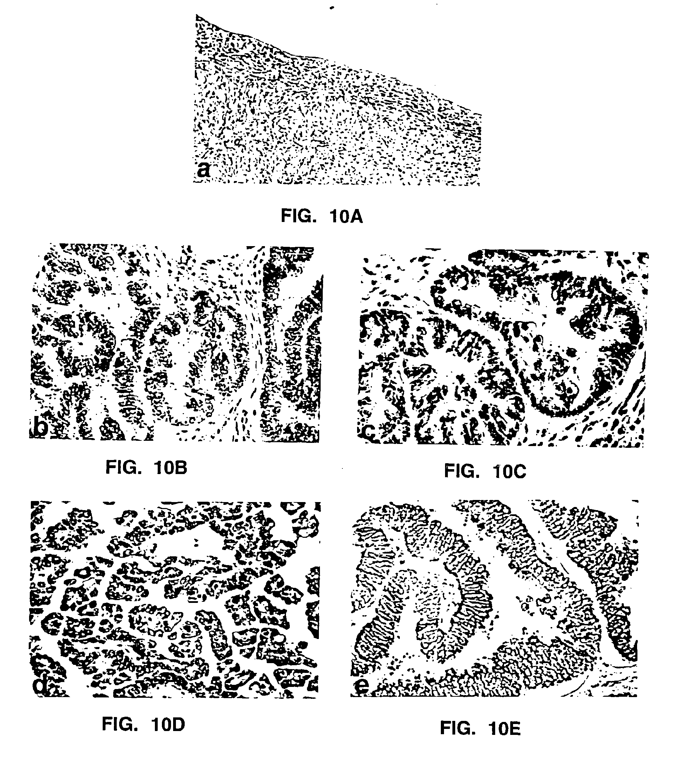

**Figure 10** shows that immunohistochemical staining of normal ovarian epithelium (**Figure 10A**) with a polyclonal antibody to a TADG-15 protease peptide shows no staining of the stroma or epithelium. However, antibody staining of carcinomas confirms the presence of TADG-15 expression in the cytoplasm of a serous low malignant potential tumor (**Figure 10B**); a mucinous low malignant potential tumor (**Figure 10C**); a serous carcinoma (**Figure 10D**); and its presence in both the cytoplasm and cell surface of an endometrioid carcinoma (**Figure 10E**).

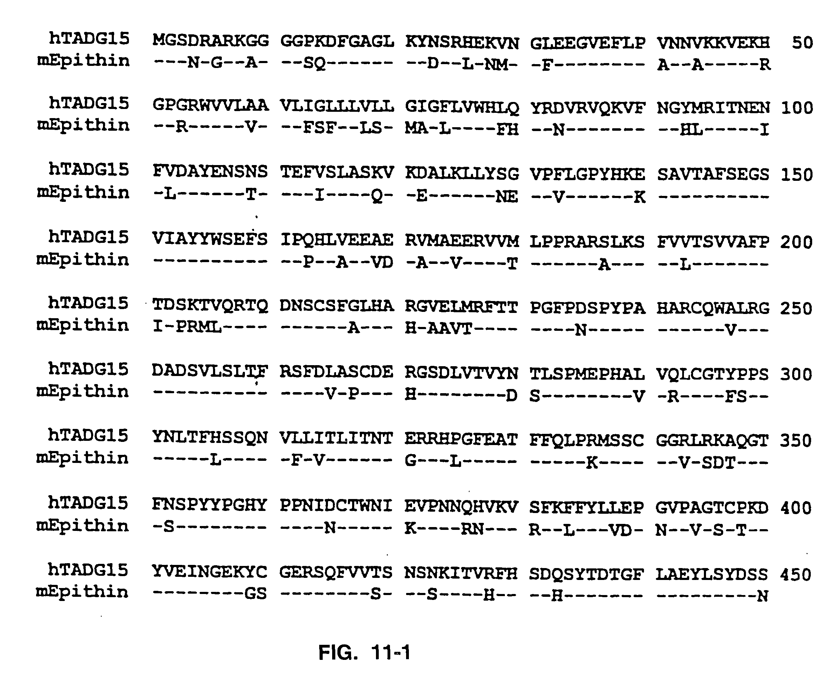

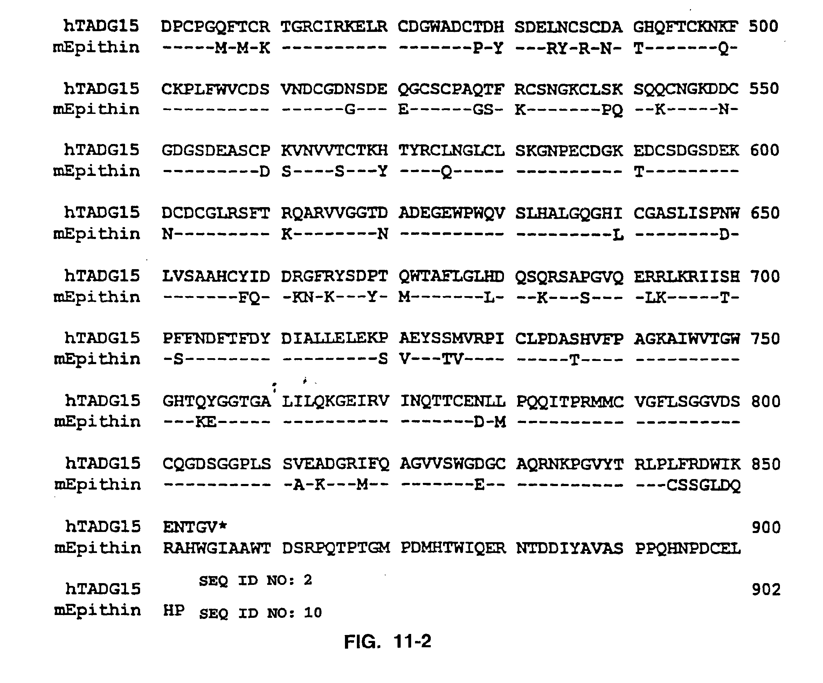

**Figure 11** shows an alignment of the human TADG15 protein sequence with that of mouse epithin which demonstrates that the proteins are 84% similar and 81 % identical over 843 amino acids. Residues that are identical between the two proteins are indicated by a "-", while the "*" symbol represents the TADG15 translation termination. The most significant difference between these two proteins lies in the carboxy-termini, which for epithin, includes 47 amino acids that are not present in TADG15.

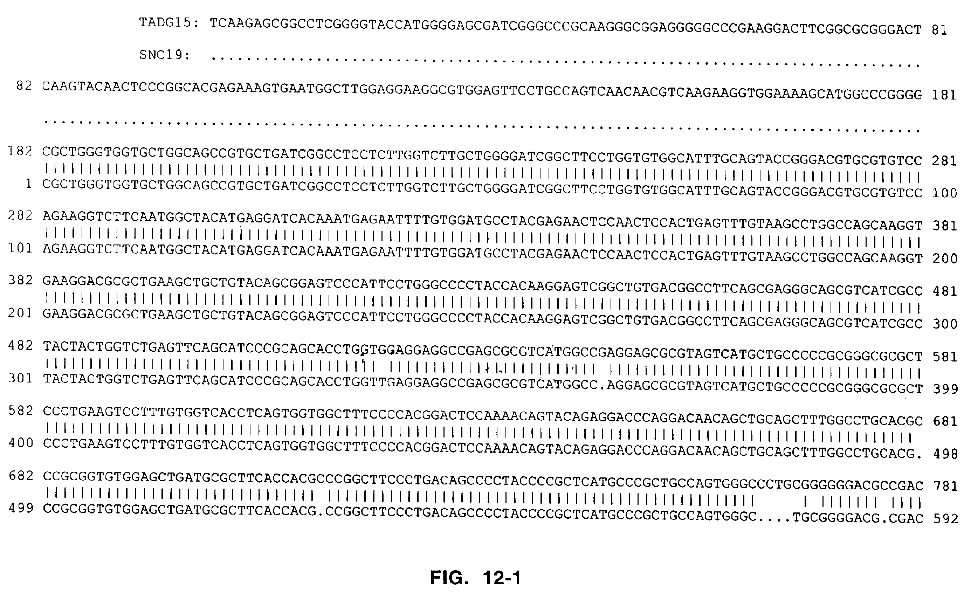

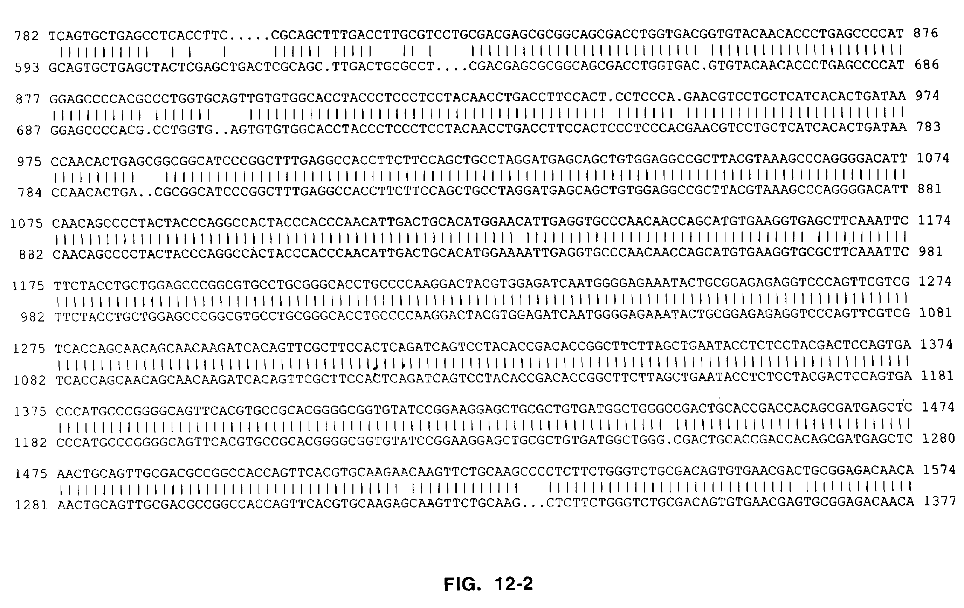

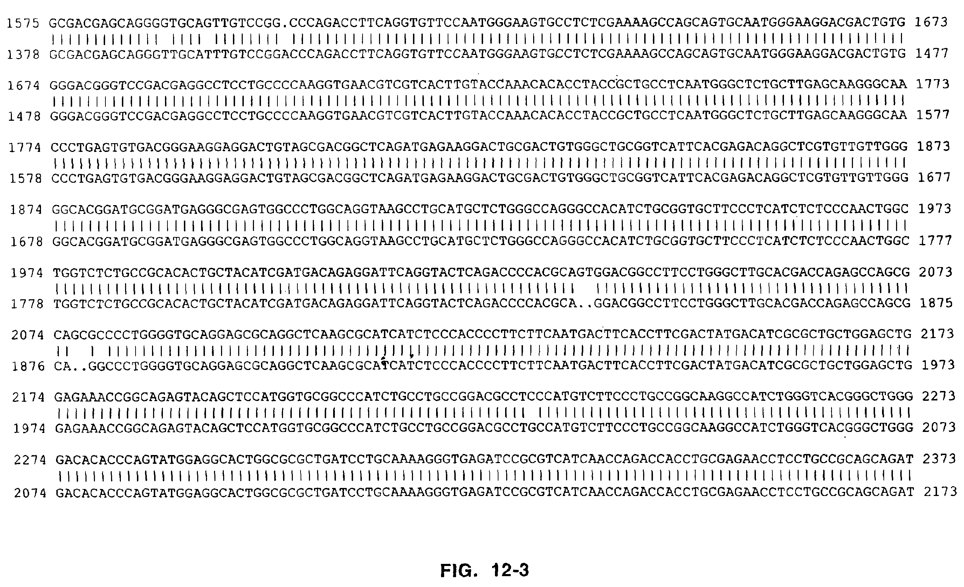

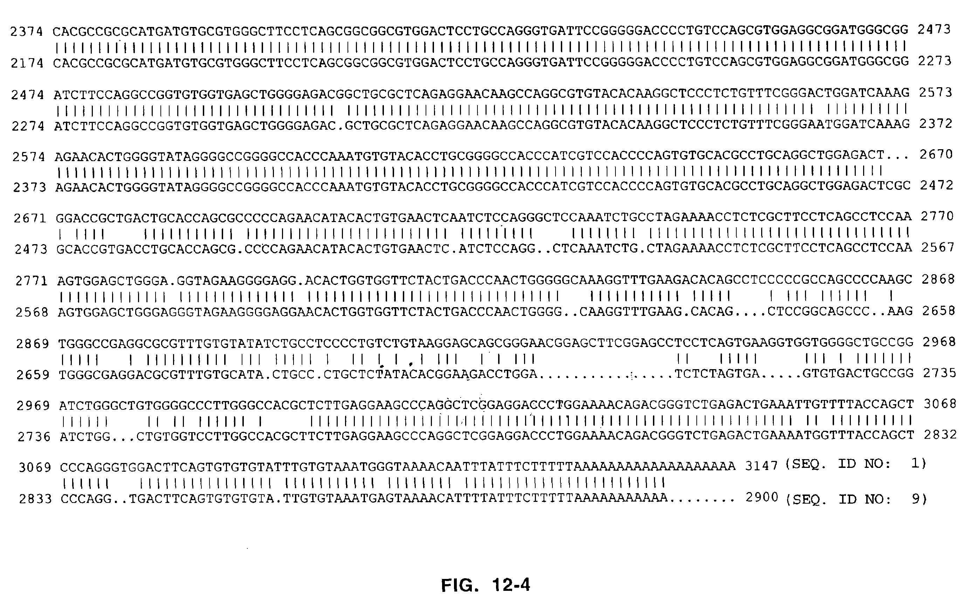

**Figure 12** shows a nucleotide sequence comparison between TADG-15 and human SNC-19 (GeneBank Accession No. #U20428).

### DETAILED DESCRIPTION OF THE INVENTION

Proteases have been implicated in the extracellular modulation required for tumor growth and invasion. In an effort to categorize those proteases contributing to ovarian carcinoma progression, redundant primers directed towards conserved amino acid domains surrounding the catalytic triad of His, Asp and Ser were utilized to amplify serine proteases differentially expressed in carcinomas. Using this method, a serine protease named TADG-15 (tumor antigen-derived gene 15) has been identified that is overexpressed in ovarian tumors. TADG-15 appears to be a transmembrane multidomain serine protease. TADG-15 is highly overexpressed in ovarian tumors based on PCR, Northern blot and immunolocalization.

The TADG-15 cDNA is 3147 base pairs long (SEQ ID No. 1) encoding for a 855 amino acid protein (SEQ ID No. 2). The availability of the TADG-15 gene provides numerous utilities. For example, the TADG-15 gene can be used as a diagnostic or therapeutic target in ovarian and other carcinomas, including breast, prostate, lung and colon.

The present invention is directed to DNA encoding a tumor antigen-derived gene (TADG-15) protein, selected from the following: (a) an isolated DNA which encodes a TADG-15 protein; (b) an isolated DNA which hybridizes under high stringency conditions to the isolated DNA of (a) above and which encodes a TADG-15 protein; and (c) an isolated DNA differing from the isolated DNAs of (a) and (b) above in codon sequence due to the degeneracy of the genetic code, and which encodes a TADG-15 protein. It is preferred that the DNA has the sequence shown in SEQ ID No. 1 and the TADG-15 protein has the amino acid sequence shown in SEQ ID No. 2.

The present invention is directed toward a vector comprising the TADG-15 DNA and regulatory elements necessary for expression of the DNA in a cell, or a vector in which the TADG-15 DNA is positioned in reverse orientation relative to the regulatory elements such that TADG-15 antisense mRNA is produced. Generally, the DNA encodes a TADG-15 protein having the amino acid sequence shown in SEQ ID No. 2. The invention is also directed toward host cells transfected with either of the above-described vector(s). Representative host cells are bacterial cells, mammalian cells, plant cells and insect cells. Preferably, the bacterial cell is *E. coli.*

The present invention is directed toward an isolated and purified TADG-15 protein coded for by DNA selected from the following: (a) an isolated DNA which encodes a TADG-15 protein; (b) an isolated DNA which hybridizes under high stringency conditions to isolated DNA of (a) above and which encodes a TADG-15 protein; and (c) an isolated DNA differing from the isolated DNAs of (a) and (b) above in codon sequence due to the degeneracy of the genetic code, and which encodes a TADG-15 protein. Preferably, the protein has the amino acid sequence shown in SEQ ID No. 2.

The present invention is directed toward a method for detecting TADG-15 mRNA in a sample, comprising the steps of: (a) contacting a sample with a probe which is specific for TADG-15; and (b) detecting binding of the probe to TADG-15 mRNA in the sample. The present invention is also directed toward a method of detecting TADG-15 protein in a sample, comprising the steps of: (a) contacting a sample with an antibody which is specific for TADG-15 or a fragment thereof; and (b) detecting binding of the antibody to TADG-15 protein in the sample. Generally, the sample is a biological sample; preferably, the biological sample is from an individual; and typically, the individual is suspected of having cancer.

The present invention is directed toward a kit for detecting TADG-15 mRNA, comprising: an oligonucleotide probe, wherein the probe is specific for TADG-15. The kit may further comprise: a label with which to label the probe; and means for detecting the label. The present invention is additionally directed toward a kit for detecting TADG-15 protein, comprising: an antibody which is specific for TADG-15 protein or a fragment thereof. The kit may further comprise: means to detect the antibody.

The present invention is directed toward a antibody which is specific for TADG-15 protein or a fragment thereof.

The present invention is directed toward a method of screening for compounds that inhibit TADG-15, comprising the steps of: (a) contacting a sample containing TADG-15 protein with a compound; and (b) assaying for TADG-15 protease activity. Typically, a decrease in the TADG-15 protease activity in the presence of the compound relative to TADG-15 protease activity in the absence of the compound is indicative of a compound that inhibits TADG-15.

The present invention is directed toward a method of inhibiting expression of TADG-15 in a cell, comprising the step of: (a) introducing a vector expressing TADG-15 antisense mRNA into a cell, which hybridizes to endogenous TADG-15 mRNA, thereby inhibiting expression of TADG-15 in the cell. Generally, the inhibition of TADG-15 expression is for treating cancer.

The present invention is directed toward a method of inhibiting a TADG-15 protein in a cell, comprising the step of: (a) introducing an antibody specific for a TADG-15 protein or a fragment thereof into a cell, which inhibits the TADG-15 protein. Generally, the inhibition of the TADG-15 protein is for treating cancer.

The present invention is directed toward a method of targeted therapy to an individual, comprising the step of: (a) administering a compound having a targeting moiety and a therapeutic moiety to an individual, wherein the targeting moiety is specific for TADG-15. Representative targeting moiety are an antibody specific for TADG-15 and a ligand or ligand binding domain (*e.g.*, CUB, LDLR, protease and extracellular) that binds TADG-15. Likewise, a representative therapeutic moiety is a radioisotope, a toxin, a chemotherapeutic agent and immune stimulants. Typically, the above-described method is useful when the individual suffers from ovarian cancer, breast cancer or cancers of the prostate, lung, colon and cervix.

The present invention is directed toward a method of diagnosing cancer in an individual, comprising the steps of: (a) obtaining a biological sample from an individual; and (b) detecting TADG-15 in the sample. Generally, the presence of TADG-15 in the sample is indicative of the presence of carcinoma in the individual, and the absence of TADG-15 in the sample is indicative of the absence of carcinoma in the individual. Generally, the biological sample is blood, ascites fluid, urine, tears, saliva or interstitial fluid. Typical means of detecting TADG-15 are by Northern blot, Western blot, PCR, dot blot, ELIZA, radioimmunoassay, DNA chips or tumor cell labeling. This method may be useful in diagnosing cancers such as ovarian, breast and other cancers in which TADG-15 is overexpressed, such as lung, prostate and colon cancers.

The present invention is also directed to an antisense oligonucleotide having the nucleotide sequence complementary to a TADG-15 mRNA sequence. The present invention is also directed to a composition comprising such an antisense oligonucleotide according and a physiologically acceptable carrier therefore.

The present invention is also directed to a method of treating a neoplastic state in an individual syndrome in an individual in need of such treatment, comprising the step of administering to said individual an effective dose of an antisense oligonucleotide of. Preferably, the neoplastic state is selected from the group consisting of from ovarian cancer, breast cancer, lung cancer, prostate cancer, colon cancer and other cancers in which TADG-15 is overexpressed. For such therapy, the oligonucleotides alone or in combination with other antineoplastic agents can be formulated for a variety of modes of administration, including systemic, topical or localized administration. Techniques and formulations generally can be found in Remington's Pharmaceutical Sciences, Mack Publishing Co., Easton, Pa. The oligonucleotide active ingredient is generally combined with a pharamceutically accceptable carrier such as a diluent or excipient which can include fillers, extenders, binders, wetting agents, disintergrants, surface active agents or lubricants, depending on the nature of the mode of administration and dosage forms. Typical dosage forms include tablets, powders, liquid preparations including suspensions, emulsions, and solutions, granules, capsules and suppositories, as well as liquid preparations for injections, including liposome preparations.

For systemic administration, injection is preferred, including intramuscular, intravenous, intraperitoneal and subcutaneous. For injection, the oligonucleotides of the invention are formulated in liquid solutions, preferably in physiologically compatible buffers. In addition, the oligonucleotides can be formulated in solid form and redissolved or suspended immediately prior to use. Lyophilized forms are also incldued. Dosages that can be used for systemic administration preferably range from about 0.01 mg/kg to 50 mg/kg administered once or twice per day. However, different dosing schedules can be utilized depending on (1) the potency of an individual oligonucleotide at inhibiting the activity of its target DNA, (2) the severity or extent of the pathological disease state, or (3) the pharmacokinetic behavior of a given oligonucleotide.

The present invention is directed toward a method of vaccinating an individual against TADG-15 overexpression, comprising the steps of: (a) inoculating an individual with a TADG-15 protein or fragment thereof which lacks TADG-15 protease activity. The inoculation with the TADG-15 protein or fragment thereof elicits an immune response in the individual, thereby vaccinating the individual against TADG-15. The vaccination with TADG-15 described herein is intended for an individual who has cancer, is suspected of having cancer or is at risk of getting cancer. Generally, the TADG-15 fragment useful for vaccinating an individual are 9-residue fragments up to 20-residue fragments, with preferred 9-residue fragments shown in SEQ ID Nos. 2, 19, 20, 21, 29, 39, 49, 50, 59, 79, 80, 81, 82, 83 , 84, 89 and 90.

The present invention is directed toward a method of producing immune-activated cells directed toward TADG-15, comprising the steps of: exposing dendritic cells to a TADG-15 protein or fragment thereof that lacks TADG-15 protease activity, wherein exposure to the TADG-15 protein or fragment thereof activates the dendritic cells, thereby producing immune-activated cells directed toward TADG-15. Representative immune-activated cells are B-cells, T-cells and dendrites. Generally, the TADG-15 fragment is a 9-residue fragment up to a 20-residue fragment, with preferable 9-residue fragments shown in SEQ ID Nos. 2, 19, 20, 21, 29, 39, 49, 50, 59, 79, 80, 81, 82, 83, 84, 89 and 90. Preferably, the dendritic cells are isolated from an individual prior to exposure, and the activated dendritic cells reintroduced into the individual subsequent to exposure. Typically, the individual for which this method may apply has cancer, is suspected of having cancer or is at risk of getting cancer.

The present invention is directed toward an immunogenic composition, comprising an immunogenic fragment of a TADG-15 protein and an appropriate adjuvant. Generally, the fragment is a 9-residue fragment up to a 20-residue fragment, with preferred 9-residue fragments shown in SEQ ID Nos. 2, 19, 20, 21, 29, 39, 49, 50, 59, 79, 80, 81, 82, 83, 84, 89 and 90.

In accordance with the present invention there may be employed conventional molecular biology, microbiology, and recombinant DNA techniques within the skill of the art. Such techniques are explained fully in the literature. See, *e.g.*, Maniatis, Fritsch & Sambrook, "Molecular Cloning: A Laboratory Manual (1982); "DNA Cloning: A Practical Approach," Volumes I and II (D.N. Glover ed. 1985); "Oligonucleotide Synthesis" (M.J. Gait ed. 1984); "Nucleic Acid Hybridization" (B.D. Hames & S.J. Higgins eds. 1985); "Transcription and Translation" (B.D. Hames & S.J. Higgins eds. 1984); "Animal Cell Culture" (R.I. Freshney, ed. 1986); "Immobilized Cells And Enzymes" (IRL Press, 1986); B. Perbal, "A Practical Guide To Molecular Cloning" (1984).

Therefore, if appearing herein, the following terms shall have the definitions set out below.

As used herein, the term "cDNA" shall refer to the DNA copy of the mRNA transcript of a gene.

As used herein, the term "derived amino acid sequence" shall mean the amino acid sequence determined by reading the triplet sequence of nucleotide bases in the cDNA.

As used herein the term "screening a library" shall refer to the process of using a labeled probe to check whether, under the appropriate conditions, there is a sequence complementary to the probe present in a particular DNA library. In addition, "screening a library" could be performed by PCR.

As used herein, the term "PCR" refers to the polymerase chain reaction that is the subject of U.S. Patent Nos. 4,683,195 and 4,683,202 to Mullis, as well as other improvements now known in the art.

The amino acid described herein are preferred to be in the "L" isomeric form. However, residues in the "D" isomeric form can be substituted for any L-amino acid residue, as long as the desired functional property of immunoglobulin-binding is retained by the polypeptide. NH₂ refers to the free amino group present at the amino terminus of a polypeptide. COOH refers t o the free carboxy group present at the carboxy terminus of a polypeptide. In keeping with standard polypeptide nomenclature, J Biol. Chem., 243:3552-59 (1969), abbreviations for amino acid residues are used as in customary in the art.

It should be noted that all amino-acid residue sequences are represented herein by formulae whose left and right orientation is in the conventional direction of amino-terminus to carboxy-terminus. Furthermore, it should be noted that a dash at the beginning or end of an amino acid residue sequence indicates a peptide bond to a further sequence of one or more amino-acid residues.

A "replicon" is any genetic element (*e.g.,* plasmid, chromosome, virus) that functions as an autonomous unit of DNA replication *in vivo; i.e.,* capable of replication under its own control.

A "vector" is a replicon, such as plasmid, phage or cosmid, to which another DNA segment may be attached so as to bring about the replication of the attached segment. A "vector" may further be defined as a replicable nucleic acid construct, *e.g.,* a plasmid or viral nucleic acid.

A "DNA molecule" refers to the polymeric form of deoxyribonucleotides (adenine, guanine, thymine, or cytosine) in its either single-stranded form or as a double-stranded helix. This term refers only to the primary and secondary structure of the molecule, and does not limit it to any particular tertiary forms. Thus, this term includes double-stranded DNA found, *inter alia,* in linear DNA molecules (*e.g.,* restriction fragments), viruses, plasmids, and chromosomes. The structure is discussed herein according to the normal convention of giving only the sequence in the 5' to 3' direction along the nontranscribed strand of DNA (*i.e.,* the strand having a sequence homologous to the mRNA).

An expression vector is a replicable construct in which a nucleic acid sequence encoding a polypeptide is operably linked to suitable control sequences capable of effecting expression of the polypeptide in a cell. The need for such control sequences will vary depending upon the cell selected and the transformation method chosen. Generally, control sequences include a transcriptional promoter and/or enhancer, suitable mRNA ribosomal binding sites, and sequences which control the termination of transcription and translation. Methods which are well known to those skilled in the art can be used to construct expression vectors containing appropriate transcriptional and translational control signals. See, for example, techniques described in Sambrook et al., 1989, Molecular Cloning: A Laboratory Manual (2nd Ed.), Cold Spring Harbor Press, N.Y. A gene and its transcription control sequences are defined as being "operably linked" if the transcription control sequences effectively control transcription of the gene. Vectors of the invention include, but are not limited to, plasmid vectors and viral vectors. Preferred viral vectors of the invention are those derived from retroviruses, adenovirus, adeno-associated virus, SV40 virus, or herpes viruses. In general, expression vectors contain promoter sequences which facilitate the efficient transcription of the inserted DNA fragment and are used in connection with the host. The expression vector typically contains an origin of replication, promoter(s), terminator(s), as well as specific genes which are capable of providing phenotypic selection in transformed cells. The transformed hosts can be fermented and cultured according to means known in the art to achieve optimal cell growth.

An "origin of replication" refers to those DNA sequences that participate in DNA synthesis.

A DNA "coding sequence" is a double-stranded DNA sequence which is transcribed and translated into a polypeptide *in vivo* when placed under the control of appropriate regulatory sequences. The boundaries of the coding sequence are determined by a start codon at the 5' (amino) terminus and a translation stop codon at the 3' (carboxyl) terminus. A coding sequence can include, but is not limited to, prokaryotic sequences, cDNA from eukaryotic mRNA, genomic DNA sequences from eukaryotic (*e.g.*, mammalian) DNA, and even synthetic DNA sequences. A polyadenylation signal and transcription termination sequence will usually be located 3' to the coding sequence.

Transcriptional and translational control sequences are DNA regulatory sequences, such as promoters, enhancers, polyadenylation signals, terminators, and the like, that provide for the expression of a coding sequence in a host cell.

A "promoter sequence" is a DNA regulatory region capable of binding RNA polymerase in a cell and initiating transcription of a downstream (3' direction) coding sequence. For purposes of defining the present invention, the promoter sequence is bounded at its 3' terminus by the transcription initiation site and extends upstream (5' direction) to include the minimum number of bases or elements necessary to initiate transcription at levels detectable above background. Within the promoter sequence will be found a transcription initiation site, as well as protein binding domains (consensus sequences) responsible for the binding of RNA polymerase. Eukaryotic promoters often, but not always, contain "TATA" boxes and "CAT" boxes. Prokaryotic promoters typically contain Shine-Dalgarno ribosome-binding sequences in addition to the -10 and -35 consensus sequences.

An "expression control sequence" is a DNA sequence that controls and regulates the transcription and translation of another DNA sequence. A coding sequence is "under the control" of transcriptional and translational control sequences in a cell when RNA polymerase transcribes the coding sequence into mRNA, which is then translated into the protein encoded by the coding sequence.

A "signal sequence" can be included near the coding sequence. This sequence encodes a signal peptide, N-terminal to the polypeptide, that communicates to the host cell to direct the polypeptide to the cell surface or secrete the polypeptide into the media, and this signal peptide is clipped off by the host cell before the protein leaves the cell. Signal sequences can be found associated with a variety of proteins native to prokaryotes and eukaryotes.

As used herein, the terms "restriction endonucleases" and "restriction enzymes" refer to enzymes, each of which cut double-stranded DNA at or near a specific nucleotide sequence.

A cell has been "transformed" by exogenous or heterologous DNA when such DNA has been introduced inside the cell. The transforming DNA may or may not be integrated (covalently linked) into the genome of the cell. In prokaryotes, yeast, and mammalian cells for example, the transforming DNA may be maintained on an episomal element such as a plasmid. With respect to eukaryotic cells, a stably transformed cell is one in which the transforming DNA has become integrated into a chromosome so that it is inherited by daughter cells through chromosome replication. This stability is demonstrated by the ability of the eukaryotic cell to establish cell lines or clones comprised of a population of daughter cells containing the transforming DNA. A "clone" is a population of cells derived from a single cell or ancestor by mitosis. A "cell line" is a clone of a primary cell that is capable of stable growth *in vitro* for many generations.

Two DNA sequences are "substantially homologous" when at least about 75% (preferably at least about 80%, and most preferably at least about 90% or 95%) of the nucleotides match over the defined length of the DNA sequences. Sequences that are substantially homologous can be identified by comparing the sequences using standard software available in sequence data banks, or in a Southern hybridization experiment under, for example, stringent conditions as defined for that particular system. Defining appropriate hybridization conditions is within the skill of the art. See, *e.g.*, Maniatis et al., *supra;* DNA Cloning, Vols. I & II, *supra*; Nucleic Acid Hybridization, *supra.*

A "heterologous" region of the DNA construct is an identifiable segment of DNA within a larger DNA molecule that is not found in association with the larger molecule in nature. Thus, when the heterologous region encodes a mammalian gene, the gene will usually be flanked by DNA that does not flank the mammalian genomic DNA in the genome of the source organism. In another example, coding sequence is a construct where the coding sequence itself is not found in nature (*e.g.*, a cDNA where the genomic coding sequence contains introns, or synthetic sequences having codons different than the native gene). Allelic variations or naturally-occurring mutational events do not give rise to a heterologous region of DNA as defined herein.

The labels most commonly employed for these studies are radioactive elements, enzymes, chemicals which fluoresce when exposed to ultraviolet light, and others. A number of fluorescent materials are known and can be utilized as labels. These include, for example, fluorescein, rhodamine, auramine, Texas Red, AMCA blue and Lucifer Yellow. A particular detecting material is anti-rabbit antibody prepared in goats and conjugated with fluorescein through an isothiocyanate. Proteins can also be labeled with a radioactive element or with an enzyme. The radioactive label can be detected by any of the currently available counting procedures. The preferred isotope may be selected from ³H, ¹⁴C, ³²P, ³⁵S, ³⁶Cl, ⁵¹Cr, ⁵⁷Co, ⁵⁸Co, ⁵⁹Fe, ⁹⁰Y, ¹²⁵I, ¹³¹I, and ¹⁸⁶Re. Enzyme labels are likewise useful, and can be detected by any of the presently utilized colorimetric, spectrophotometric, fluorospectrophotometric, amperometric or gasometric techniques. The enzyme is conjugated to the selected particle by reaction with bridging molecules such as carbodiimides, diisocyanates, glutaraldehyde and the like. Many enzymes which can be used in these procedures are known and can be utilized. The preferred are peroxidase, β-glucuronidase, β-D-glucosidase, β-D-galactosidase, urease, glucose oxidase plus peroxidase and alkaline phosphatase. U.S. Patent Nos. 3,654,090, 3,850,752, and 4,016,043 are referred to by way of example for their disclosure of alternate labeling material and methods.

A particular assay system developed and utilized in the art is known as a receptor assay. In a receptor assay, the material to be assayed is appropriately labeled and then certain cellular test colonies are inoculated with a quantitiy of both the label after which binding studies are conducted to determine the extent to which the labeled material binds to the cell receptors. In this way, differences in affinity between materials can be ascertained.

An assay useful in the art is known as a "cis/trans" assay. Briefly, this assay employs two genetic constructs, one of which is typically a plasmid that continually expresses a particular receptor of interest when transfected into an appropriate cell line, and the second of which is a plasmid that expresses a reporter such as luciferase, under the control of a receptor/ligand complex. Thus, for example, if it is desired to evaluate a compound as a ligand for a particular receptor, one of the plasmids would be a construct that results in expression of the receptor in the chosen cell line, while the second plasmid would possess a promoter linked to the luciferase gene in which the response element to the particular receptor is inserted. If the compound under test is an agonist for the receptor, the ligand will complex with the receptor, and the resulting complex will bind the response element and initiate transcription of the luciferase gene. The resulting chemiluminescence is then measured photometrically, and dose response curves are obtained and compared to those of known ligands. The foregoing protocol is described in detail in U.S. Patent No. 4,981,784.

As used herein, the term "host" is meant to include not only prokaryotes but also eukaryotes such as yeast, plant and animal cells. A recombinant DNA molecule or gene which encodes a human TADG-15 protein of the present invention can be used to transform a host using any of the techniques commonly known to those of ordinary skill in the art. Especially preferred is the use of a vector containing coding sequences for the gene which encodes a human TADG-15 protein of the present invention for purposes of prokaryote transformation. Prokaryotic hosts may include *E. coli, S. tymphimurium, Serratia marcescens* and *Bacillus subtilis.* Eukaryotic hosts include yeasts such as *Pichia pastoris,* mammalian cells and insect cells.

The invention includes a substantially pure DNA encoding a TADG-15 protein, a DNA strand which will hybridize at high stringency to a probe containing a sequence of at least 1 5 consecutive nucleotides of (SEQ ID No. 1). The protein encoded by the DNA of this invention may share at least 80% sequence identity (preferably 85%, more preferably 90%, and most preferably 95%) with the amino acids listed in Figures 3 and 4 (SEQ ID No. 2). More preferably, the DNA includes the coding sequence of the nucleotides of Figure 2 (SEQ ID No. 1), or a degenerate variant of such a sequence. This invention also includes a substantially pure DNA containing a sequence of at least 15 consecutive nucleotides (preferably 20, more preferably 30, even more preferably 50, and most preferably all) of the region from nucleotides 1 to 3147 of the nucleotides shown in Figure 2 (SEQ ID No. 1).

By "substantially pure DNA" is meant DNA that is not part of a milieu in which the DNA naturally occurs, by virtue of separation (partial or total purification) of some or all of the molecules of that milieu, or by virtue of alteration of sequences that flank the claimed DNA. The term therefore includes, for example, a recombinant DNA which is incorporated into a vector, into an autonomously replicating plasmid or virus, or into the genomic DNA of a prokaryote or eukaryote; or which exists as a separate molecule (*e.g.*, a cDNA or a genomic or cDNA fragment produced by polymerase chain reaction (PCR) or restriction endonuclease digestion) independent of other sequences. It also includes a recombinant DNA which is part of a hybrid gene encoding additional polypeptide sequence, *e.g.*, a fusion protein. Also included is a recombinant DNA which includes a portion of the nucleotides listed in Figure 2 (SEQ ID No. 1) and which encodes an alternative splice variant of TADG-15.

By a "substantially pure protein" is meant a protein which has been separated from at least some of those components which naturally accompany it. Typically, the protein is substantially pure when it is at least 60% (by weight) free from the proteins and other naturally-occurring organic molecules with which it is naturally associated *in vivo*. Preferably, the purity of the preparation (by weight) is at least 75%, more preferably at least 90%, and most preferably at least 99%. A substantially pure TADG-15 protein may be obtained, for example, by extraction from a natural source; by expression of a recombinant nucleic acid encoding a TADG-15 polypeptide; or by chemically synthesizing the protein. Purity can be measured by any appropriate method, *e*.*g*., column chromatography, such as immunoaffinity chromatography using an antibody specific for TADG-15, polyacrylamide gel electrophoresis, or HPLC analysis. A protein is substantially free of naturally associated components when it is separated from at least some of those contaminants which accompany it in its natural state. Thus, a protein which is chemically synthesized or produced in a cellular system different from the cell from which it naturally originates will be, by definition, substantially free from its naturally associated components. Accordingly, substantially pure proteins include eukaryotic proteins synthesized in *E. coli,* other prokaryotes, or any other organism in which they do not naturally occur.

The term "oligonucleotide", as used herein, is defined as a molecule comprised of two or more ribonucleotides, preferably more than three. Its exact size will depend upon many factors, which, in turn, depend upon the ultimate function and use of the oligonucleotide. The term "primer", as used herein, refers to an oligonucleotide, whether occurring naturally (as in a purified restriction digest) or produced synthetically, and which is capable of initiating synthesis of a strand complementary to a nucleic acid when placed under appropriate conditions, *i*.*e*., in the presence of nucleotides and an inducing agent, such as a DNA polymerase, and at a suitable temperature and pH. The primer may be either single-stranded or double-stranded and must be sufficiently long to prime the synthesis of the desired extension product in the presence of the inducing agent. The exact length of the primer will depend upon many factors, including temperature, sequence and/or homology of primer and the method used. For example, in diagnostic applications, the oligonucleotide primer typically contains 15-25 or more nucleotides, depending upon the complexity of the target sequence, although it may contain fewer nucleotides.

The primers herein are selected to be "substantially" complementary to particular target DNA sequences. This means that the primers must be sufficiently complementary to hybridize with their respective strands. Therefore, the primer sequence need not reflect the exact sequence of the template. For example, a non-complementary nucleotide fragment (i.e., containing a restriction site) may be attached to the 5' end of the primer, with the remainder of the primer sequence being complementary to the strand. Alternatively, non-complementary bases or longer sequences can be interspersed into the primer, provided that the primer sequence has sufficient complementary with the sequence to hybridize therewith and form the template for synthesis of the extension product.

The probe to which the DNA of the invention hybridizes preferably consists of a sequence of at least 2 0 consecutive nucleotides, more preferably 40 nucleotides, even more preferably 50 nucleotides, and most preferably 100 nucleotides or more (up to 100%) of the coding sequence of the nucleotides listed in Figure 2 (SEQ ID No. 1) or the complement thereof. Such a probe is useful for detecting expression of TADG-15 in a cell by a method including the steps of (a) contacting mRNA obtained from the cell with a labeled TADG-15 hybridization probe; and (b) detecting hybridization of the probe with the mRNA.

By "high stringency" is meant DNA hybridization and wash conditions characterized by high temperature and low salt concentration, *e*.*g*., wash conditions of 65°C at a salt concentration of approximately 0.1X SSC, or the functional equivalent thereof. For example, high stringency conditions may include hybridization at about 42°C in the presence of about 50% formamide; a first wash at about 65°C with about 2X SSC containing 1% SDS; followed by a second wash at about 65°C with about 0.1X SSC.

The DNA may have at least about 70% sequence identity to the coding sequence of the nucleotides listed in Figure 2 (SEQ ID No. 1), preferably at least 75% (*e.g.,* at least 80%); and most preferably at least 90%. The identity between two sequences is a direct function of the number of matching or identical positions. When a position in both of the two sequences is occupied by the same monomeric subunit, *e.g.*, if a given position is occupied by an adenine in each of two DNA molecules, then they are identical at that position. For example, if 7 positions in a sequence 10 nucleotides in length are identical to the corresponding positions in a second 10-nucleotide sequence, then the two sequences have 70% sequence identity. The length of comparison sequences will generally be at least 50 nucleotides, preferably at least 60 nucleotides, more preferably at least 75 nucleotides, and most preferably 100 nucleotides. Sequence identity is typically measured using sequence analysis software (*e.g.*, Sequence Analysis Software Package of the Genetics Computer Group (GCG), University of Wisconsin Biotechnology Center, 1710 University Avenue, Madison, WI 53705).

The present invention comprises a vector comprising a DNA sequence which encodes a human TADG-15 protein, wherein said vector is capable of replication in a host, and comprises, in operable linkage: a) an origin of replication; b) a promoter; and c) a DNA sequence coding for said TADG-15 protein. Preferably, the vector of the present invention contains a portion of the DNA sequence shown in SEQ ID No. 1. Vectors may be used to amplify and/or express nucleic acid encoding a TADG-15 protein or fragment thereof.

In addition to substantially full-length proteins, the invention also includes fragments (*e.g.*, antigenic fragments) of the TADG-15 protein (SEQ ID No. 2). As used herein, "fragment," as applied to a polypeptide, will ordinarily be at least 6 residues, more typically at least 9-12 residues, and preferably at least 13-20 residues in length, but less than the entire, intact sequence. Alternatively, a fragment may be an individual domain of 20-120 residues from SEQ ID No. 2. Fragments of the TADG-15 protein can be generated by methods known to those skilled in the art, *e.g.,* by enzymatic digestion of naturally occurring o r recombinant TADG-15 protein, by recombinant DNA techniques using an expression vector that encodes a defined fragment of TADG-15, or by chemical synthesis. The ability of a candidate fragment to exhibit a characteristic of TADG-15 (*e.g.*, binding to an antibody specific for TADG-15) can be assessed by methods described herein. Purified TADG-15 or antigenic fragments of TADG-15 can be used to generate new antibodies or to test existing antibodies (*e.g.*, as positive controls in a diagnostic assay) by employing standard protocols known to those skilled in the art. Included in this invention is polyclonal antisera generated by using TADG-15 or a fragment of TADG-15 as the immunogen in, *e.g.*, rabbits. Standard protocols for monoclonal and polyclonal antibody production known to those skilled in this art are employed. The monoclonal antibodies generated by this procedure can be screened for the ability to identify recombinant TADG-15 cDNA clones, and to distinguish them from other cDNA clones.

Further included in this invention are TADG-15 proteins which are encoded, at least in part, by portions of SEQ ID No. 2, *e.g.*, products of alternative mRNA splicing or alternative protein processing events, or in which a section of TADG-15 sequence has been deleted. The fragment, or the intact TADG-15 polypeptide, may be covalently linked to another polypeptide, *e.g.,* one which acts as a label, a ligand or a means to increase antigenicity.

The invention also includes a polyclonal or monoclonal antibody which specifically binds to TADG-15. The invention encompasses not only an intact monoclonal antibody, but also an immunologically-active antibody fragment, *e.g.,* a Fab or (Fab)₂ fragment; an engineered single chain Fv molecule; or a chimeric molecule, *e.g.*, an antibody which contains the binding specificity of one antibody, *e.g.,* of murine origin, and the remaining portions of another antibody, *e.g.*, of human origin.

In one embodiment, the antibody, or a fragment thereof, may be linked to a toxin or to a detectable label, *e.g.,* a radioactive label, non-radioactive isotopic label, fluorescent label, chemiluminescent label, paramagnetic label, enzyme label, or colorimetric label. Examples of suitable toxins include diphtheria toxin, *Pseudomonas* exotoxin A, ricin, and cholera toxin. Examples of suitable enzyme labels include malate hydrogenase, staphylococcal nuclease, delta-5-steroid isomerase, alcohol dehydrogenase, alpha-glycerol phosphate dehydrogenase, triose phosphate isomerase, peroxidase, alkaline phosphatase, asparaginase, glucose oxidase, beta-galactosidase, ribonuclease, urease, catalase, glucose-6-phosphate dehydrogenase, glucoamylase, acetylcholinesterase, etc. Examples of suitable radioisotopic labels include ³H, ¹²⁵I, ¹³¹I, ³²P, ³⁵S, ¹⁴C, etc.

Paramagnetic isotopes for purposes of *in vivo* diagnosis can also be used according to the methods of this invention. There are numerous examples of elements that are useful in magnetic resonance imaging. For discussions on *in vivo* nuclear magnetic resonance imaging, see, for example, Schaefer et al., (1989) JACC 14, 472-480; Shreve et al., (1986) Magn. Reson. Med. 3, 336-340; Wolf, G. L., (1984) Physiol. Chem. Phys. Med. NMR 16, 93-95; Wesbey et al., (1984) Physiol. Chem. Phys. Med. NMR 16, 145-155; Runge et al., (1984) Invest. Radiol. 19, 408-415. Examples of suitable fluorescent labels include a fluorescein label, an isothiocyalate label, a rhodamine label, a phycoerythrin label, a phycocyanin label, an allophycocyanin label, an ophthaldehyde label, a fluorescamine label, etc. Examples of chemiluminescent labels include a luminal label, an isoluminal label, an aromatic acridinium ester label, an imidazole label, an acridinium salt label, an oxalate ester label, a luciferin label, a luciferase label, an aequorin label, etc.

Those of ordinary skill in the art will know of other suitable labels which may be employed in accordance with the present invention. The binding of these labels to antibodies or fragments thereof can be accomplished using standard techniques commonly known and used by those of ordinary skill in the art. Typical techniques are described by Kennedy et al., (1976) Clin. Chim. Acta 70, 1-31; and Schurs et al., (1977) Clin. Chim. Acta 81, 1-40. Coupling techniques mentioned in the latter are the glutaraldehyde method, the periodate method, the dimaleimide method, the m-maleimidobenzyl-N-hydroxysuccinimide ester method. All of these methods are incorporated by reference herein.

Also within the invention is a method of detecting TADG-15 protein in a biological sample, which includes the steps of contacting the sample with the labeled antibody, *e*.*g*., radioactively tagged antibody specific for TADG-15, and determining whether the antibody binds to a component of the sample. Antibodies to the TADG-15 protein can be used in an immunoassay to detect increased levels of TADG-15 protein expression in tissues suspected of neoplastic transformation. These same uses can be achieved with Northern blot assays and analyses.

As described herein, the invention provides a number of diagnostic advantages and uses. For example, the TADG-15 protein is useful in diagnosing cancer in different tissues since this protein is highly overexpressed in tumor cells. Antibodies (or antigen-binding fragments thereof) which bind to an epitope specific for TADG-15, are useful in a method of detecting TADG-15 protein in a biological sample for diagnosis of cancerous or neoplastic transformation. This method includes the steps of obtaining a biological sample (*e.g.*, cells, blood, plasma, tissue, etc.) from a patient suspected of having cancer, contacting the sample with a labeled antibody (*e.g.*, radioactively tagged antibody) specific for TADG-15, and detecting the TADG-15 protein using standard immunoassay techniques such as an ELISA. Antibody binding to the biological sample indicates that the sample contains a component which specifically binds to an epitope within TADG-15.

Likewise, a standard Northern blot assay can be used to ascertain the relative amounts of TADG-15 mRNA in a cell or tissue obtained from a patient suspected of having cancer, in accordance with conventional Northern hybridization techniques known to those of ordinary skill in the art. This Northern assay uses a hybridization probe, *e*.*g*., radiolabelled TADG-15 cDNA, either containing the full-length, single stranded DNA having a sequence complementary to SEQ ID No. 1 (Figure 2), or a fragment of that DNA sequence at least 20 (preferably at least 30, more preferably at least 50, and most preferably at least 1 00 consecutive nucleotides in length). The DNA hybridization probe can be labeled by any of the many different methods known to those skilled in this art.

Accordingly, the present invention is directed to the following embodiments:
1. DNA encoding a tumor antigen-derived gene (TADG-15) protein, selected from the group consisting of:
   (a) isolated DNA which encodes a TADG-15 protein;
   (b) isolated DNA which hybridizes under high stringency conditions to the isolated DNA of (a) above and which encodes a TADG-15 protein; and
   (c) isolated DNA differing from the isolated DNAs of (a) and (b) above in codon sequence due to the degeneracy of the genetic code, and which encodes a TADG-15 protein.
2. The DNA of claim 1, wherein said DNA has the sequence shown in SEQ ID No. 1.
3. The DNA of claim 1, wherein said TADG-15 protein has the amino acid sequence shown in SEQ ID No. 2.
4. A vector comprising the DNA of claim 1 and regulatory elements necessary for expression of said DNA in a cell.
5. The vector of claim 4, wherein said DNA encodes a TADG-15 protein having the amino acid sequence shown in SEQ ID No. 2.
6. The vector of claim 4, wherein said DNA is positioned in reverse orientation relative to said regulatory elements such that TADG-15 antisense mRNA is produced.
7. A host cell transfected with the vector of claim 4, said vector expressing a TADG-15 protein.
8. The host cell of claim 7, wherein said cell is selected from the group consisting of bacterial cells, mammalian cells, plant cells and insect cells.
9. The host cell of claim 8, wherein said bacterial cell is *E*. *coli.*
10. Isolated and purified TADG-15 protein coded for by DNA selected from the group consisting of:
   (a) isolated DNA which encodes a TADG-15 protein;
   (b) isolated DNA which hybridizes under high stringency conditions to isolated DNA of (a) above and which encodes a TADG-15 protein; and
   (c) isolated DNA differing from the isolated DNAs of (a) and (b) above in codon sequence due to the degeneracy of the genetic code, and which encodes a TADG-15 protein,
11. The TADG-15 protein of claim 10, wherein said protein has the amino acid sequence shown in SEQ ID No. 2.
12. A method for detecting TADG-15 mRNA in a sample, comprising the steps of:
   (a) contacting a sample with a probe, wherein said probe is specific for TADG-15; and
   (b) detecting binding of said probe to TADG-15 mRNA in said sample.
13. The method of claim 12, wherein said sample is a biological sample.
14. The method of claim 13, wherein said biological sample is from an individual.
15. The method of claim 14, wherein said individual is suspected of having cancer.
16. A kit for detecting TADG-15 mRNA, comprising:
   an oligonucleotide probe, wherein said probe is specific for TADG-15.
17. The kit of claim 16, further comprising:
   a label with which to label said probe; and
   means for detecting said label.
18. A method of detecting TADG-15 protein in a sample, comprising the steps of:
   (a) contacting a sample with an antibody, wherein said antibody is specific for TADG-15 or a fragment thereof; and
   (b) detecting binding of said antibody to TADG-15 protein in said sample.
19. The method of claim 18, wherein said sample is a biological sample.
20. The method of claim 19, wherein said biological sample is from an individual.
21. The method of claim 20, wherein said individual is suspected of having cancer.
22. A kit for detecting TADG-15 protein, comprising:
   an antibody, wherein said antibody is specific for TADG-15 protein or a fragment thereof.
23. The kit of claim 22, further comprising:
   means to detect said antibody.
24. An antibody, wherein said antibody is specific for TADG-15 protein or a fragment thereof.
25. A method of screening for compounds that inhibit TADG-15, comprising the steps of:
   (a) contacting a sample with a compound, wherein said sample comprises TADG-15 protein; and
   (b) assaying for TADG-15 protease activity, wherein a decrease in said TADG-15 protease activity in the presence of said compound relative to TADG-15 protease activity in the absence of said compound is indicative of a compound that inhibits TADG-15.
26. A method of inhibiting expression of TADG-15 in a cell, comprising the step of introducing the vector of claim 6 into a cell, wherein expression of said vector produces TADG-15 antisense mRNA in said cell, wherein said TADG-15 antisense mRNA hybridizes to endogenous TADG-15 mRNA, thereby inhibiting expression of TADG-15 in said cell.
27. A method of inhibiting a TADG-15 protein in a cell, comprising the step of introducing an antibody into a cell, wherein said antibody is specific for a TADG-15 protein or a fragment thereof, wherein binding of said antibody to said TADG-15 protein inhibits said TADG-15 protein.
28. A method of targeted therapy to an individual, comprising the step of:
   (a) administering a compound to an individual, wherein said compound has a targeting moiety and a therapeutic moiety, wherein said targeting moiety is specific for TADG-15.
29. The method of claim 28, wherein said targeting moiety is selected from the group consisting of an antibody specific for TADG-15 and a ligand or ligand binding domain that binds TADG-15.
30. The method of claim 28, wherein said therapeutic moiety is selected from the group consisting of a radioisotope, a toxin, a chemotherapeutic agent, an immune stimulant and a cytotoxic agent.
31. The method of claim 28, wherein said individual suffers from ovarian cancer, lung cancer, prostate cancer, colon cancer and other cancers in which TADG-15 is overexpressed.
32. A method of diagnosing cancer in an individual, comprising the steps of:
   (a) obtaining a biological sample from an individual;
   (b) detecting TADG-15 in said sample,
   wherein the presence of TADG-15 in said sample is indicative of the presence of carcinoma in said individual, wherein the absence of TADG-15 in said sample is indicative of the absence of carcinoma in said individual.
33. The method of claim 32, wherein said biological sample is selected from the group consisting of blood, urine, saliva, tears, interstitial fluid, ascites fluid, tumor tissue biopsy and circulating tumor cells.
34. The method of claim 32, wherein said detection of said TADG-15 is by means selected from the group consisting of Northern blot, Western blot, PCR, dot blot, ELIZA sandwich assay, radioimmunoassay, DNA array chips and flow cytometry.
35. The method of claim 32, wherein said carcinoma is selected from the group consisting of ovarian, breast, lung, colon, prostate and others in which TADG-15 is overexpressed.
36. A method of vaccinating an individual against TADG-15, comprising the steps of:
   inoculating an individual with a TADG-15 protein or fragment thereof, wherein said TADG-15 protein or fragment thereof lacks TADG-15 protease activity, wherein said inoculation with said TADG-15 protein or fragment thereof elicits an immune response in said individual, thereby vaccinating said individual against TADG-15.
37. The method of claim 36, wherein said individual has cancer, is suspected of having cancer or is at risk of getting cancer.
38. The method of claim 36, wherein said TADG-15 fragment is selected from the group consisting of a 9-residue fragment up to a 20-residue fragment.
39. The method of claim 38, wherein said 9-residue fragment is selected from the group consisting of SEQ ID Nos. 2, 19, 20, 21, 29, 39, 49, 50, 59, 79, 80, 81, 82, 83, 84, 89 and 90.
40. A method of producing immune-activated cells directed toward TADG-15, comprising the steps of:
   exposing dendritic cells to a TADG-15 protein or fragment thereof, wherein said TADG-15 protein or fragment thereof lacks TADG-15 protease activity, wherein said exposure to said TADG-15 protein or fragment thereof activates said dendritic cells, thereby producing immune-activated cells directed toward TADG-15.
41. The method of claim 40, wherein said immune-activated cells are selected from the group consisting of B-cells, T-cells and dendrites.
42. The method of claim 40, wherein said TADG-15 fragment is selected from the group consisting of a 9-residue fragment up to a 20-residue fragment.
43. The method of claim 42, wherein said 9-residue fragment is selected from the group consisting of SEQ ID Nos. 2, 19, 20, 21, 29, 39, 49, 50, 59, 79, 80, 81, 82, 83, 84, 89 and 90.
44. The method of claim 40, wherein said dendritic cells are isolated from an individual prior to said exposure, wherein said activated dendritic cells are reintroduced into said individual subsequent to said exposure.
45. The method of claim 44, wherein said individual has cancer, is suspected of having cancer or is at risk of getting cancer.
46. An immunogenic composition, comprising an immunogenic fragment of a TADG-15 protein and an appropriate adjuvant.
47. The immunogenic composition of claim 46, wherein said fragment is selected from the group consisting of a 9-residue fragment up to a 20-residue fragment.
48. The immunogenic composition of claim 47, wherein said 9-residue fragment is selected from the group consisting of SEQ ID Nos. 2, 19, 20, 21, 29, 39, 49, 50, 59, 79, 80, 81, 82, 83, 84, 89 and 90.
49. An oligonucleotide having the nucleotide sequence complementary to a sequence of claim 1.
50. A composition comprising the oligonucleotide according to claim 49 and a physiologically acceptable carrier therefore.
51. A method of treating a neoplastic state in a n individual syndrome in an individual in need of such treatment, comprising the step of administering to said individual an effective dose of the oligonucleotide of claim 49.
52. The method of claim 51, wherein said neoplastic state is selected from the group consisting of ovarian cancer, breast cancer, lung cancer, colon cancer, prostate cancer and other cancers in which TADG-15 is overexpressed.

The following examples are given for the purpose of illustrating various embodiments of the invention and are not meant to limit the present invention in any fashion.

### EXAMPLE 1

### Tissue collection and storage

Upon patient hysterectomy, bilateral salpingo-oophorectomy, or surgical removal of neoplastic tissue, the specimen is retrieved and placed on ice. The specimen was then taken to the resident pathologist for isolation and identification of specific tissue samples. Finally, the sample was frozen in liquid nitrogen, logged into the laboratory record and stored at -80°C.

Additional specimens were frequently obtained from the Cooperative Human Tissue Network (CHTN). These samples were prepared by the CHTN and shipped on dry ice. Upon arrival, these specimens (*e.g.*, blood (serum), urine, saliva, tears and insterstitial fluid) were logged into the laboratory record and stored at -80°C. Participation of the following divisions of the Cooperative Human Tissue Network (CHTN) in providing tumor tissues is acknowledged: Western Division, Case Western Reserve University, (Cleveland, OH); Midwestern Division, Ohio state University, (Columbus, OH); Eastern Division, NDRI, (Philadelphia, PA); Pediatric Division, Children's Hospital, (Columbus, OH); Southern Division, University of Alabama at Birmingham, (Birmingham, AL).

### EXAMPLE 2

### mRNA isolation and cDNA synthesis

Forty-one ovarian tumors (10 low malignant potential tumors and 31 carcinomas) and 10 normal ovaries were obtained from surgical specimens and frozen in liquid nitrogen. The human ovarian carcinoma cell lines SW626 and CAOV3, and the human breast carcinoma cell lines MDA-MB-231 and MDA-MB-435S, were purchased from the American Type Culture Collection (Rockville, MD). Cells were cultured to sub-confluency in Dulbecco's modified Eagle's medium supplemented with 10% (v/v) fetal bovine serum and antibiotics.

Messenger RNA (mRNA) isolation was performed according to the manufacturer's instructions using the Mini RiboSep™ Ultra mRNA Isolation Kit purchased from Becton Dickinson. In this procedure, poly A⁺ mRNA was isolated directly from the tissue lysate using the affinity chromatography media oligo(dT) cellulose. The amount of mRNA recovered was quantitated by UV spectrophotometry.

First-strand complementary DNA (cDNA) was synthesized using 5.0 µg of mRNA and either random hexamer or oligo(dT) primers according to the manufacturer's protocol utilizing a first strand synthesis kit obtained from CLONTECH (Palo Alto, CA). The purity of the cDNA was evaluated by PCR using primers specific for the p53 gene. These primers span an intron such that pure cDNA can be distinguished from cDNA that is contaminated with genomic DNA.

### EXAMPLE 3

### PCR with redundant primers, cloning of TADG-15 cDNA, T-vector ligation and transformations and DNA sequencing

Redundant primers,
forward 5'-TGGGTIGTIACIGCIGCICA(C/T)TG-3' (SEQ ID No. 11) and reverse 5'-A(A/G)IGGICCICCI(C/G)(T/A)(A/G)TCICC-3' (SEQ ID No. 12), corresponding to the amino acids surrounding the catalytic triad for serine proteases, were used to compare the PCR products from normal and carcinoma cDNAs.

The purified PCR products were ligated into the Promega T-vector plasmid and the ligation products used to transform JM109 competent cells according to the manufacturer's instructions (Promega). Positive colonies were cultured for amplification, the plasmid DNA isolated using the Wizard™ Minipreps DNA purification system (Promega), and the plasmids were digested with *Apa*I and *Sac*I restriction enzymes to determine the size of the insert. Plasmids with inserts of the size(s) visualized by the previously described PCR product gel electrophoresis were sequenced.

Individual colonies were cultured and plasmid DNA was isolated using the Wizard Miniprep DNA purification system (Promega). Applied Biosystems Model 373A DNA sequencing system was used for direct cDNA sequence determination. Utilizing a plasmid-specific primer near the cloning site, sequencing reactions were carried out using PRISM™ Ready Reaction Dye Deoxy™ terminators (Applied Biosystems) according to the manufacturer's instructions. Residual dye terminators were removed from the completed sequencing reaction using a Centri-sep™ spin column (Princeton Separation). Based upon the determined sequence, primers that specifically amplify the gene of interest were designed and synthesized.

The original TADG-15 subclone (436bp) was randomly labeled and used as a probe to screen an ovarian tumor cDNA library by standard hybridization techniques.¹³ The library was constructed in 8ZAP using mRNA isolated from the tumor cells of a stage III/grade III ovarian adenocarcinoma patient. Three overlapping clones were obtained which spanned 3147 nucleotides.

### EXAMPLE 4

### Northern blot analysis

10 µg mRNAs were size separated by electrophoresis through a 1% formaldehyde-agarose gel in 0.02 M MOPS, 0.05 M sodium acetate (pH 7.0), and 0.001 M EDTA. The mRNAs were then blotted to Hybond-N⁺ nylon membrane (Amersham) by capillary action in 20x SSPE. The RNAs are fixed to the membrane by baking for 2 hours at 80°C. ³²P-labeled cDNA probes were made by Prime-a-Gene Labeling System (Promega). The PCR products amplified by the same primers described above were used for probes. The blots were prehybridized for 30 min and hybridized for 60 min at 68°C with ³²P-labeled cDNA probe in ExpressHyb Hybridization Solution (CLONTECH). Control hybridization to determine relative gel loading was performed with a β-tubulin probe.

Normal human tissues; spleen, thymus, prostate, testis, ovary, small intestine, colon and peripheral blood leukocyte, and normal human fetal tissues; brain, lung, liver and kidney (Human Multiple Tissue Northern Blot; CLONTECH) were also examined by the same hybridization procedure. Additional multiple tissue northern (MTN) blots from CLONTECH include the Human MTN blot, the Human MTN II blot, the Human Fetal MTN II blot, and the Human Brain MTN III blot.

### EXAMPLE 5

### Western blot analysis

Polyclonal rabbit antibody was generated by immunization with a poly-lysine linked multiple Ag peptide derived from the TADG-15 protein sequence 'LFRDWIKENTGV' (SEQ ID No. 13). Approximately 20 µg of cell lysates were separated on a 15% SDS-PAGE gel and electroblotted to PVDF at 100 V for 40 min at 4°C. The proteins were fixed to the membrane by incubation in 50% MeOH for 10 min. The membrane was blocked overnight in TBS (pH 7.8) containing 0.2% non-fat milk. Primary antibody was added to the membrane at a dilution of 1:100 in 0.2% milk/TBS and incubated for 2 h at room temperature. The blot was washed and incubated with a 1:3000 dilution of alkaline-phosphatase conjugated goat anti-rabbit IgG (BioRad) for 1 h at room temperature. The blot was washed and incubated with a chemiluminescent substrate before a 10 sec exposure to X-ray film for visualization.

### EXAMPLE 6

### Quantitative PCR

The mRNA overexpression of TADG-15 was determined using a quantitative PCR. Quantitative PCR was performed.^{11,12} Oligonucleotide primers were used for TADG-15:
forward 5'-ATGACAGAGGATTCAGGTAC-3' (SEQ ID No. 14) and
reverse 5'-GAAGGTGAAGTCATTGAAGA-3' (SEQ ID No. 15); and and for β-tubulin:
forward 5'-CGCATCAACGTGTACTACAA-3' (SEQ ID No. 16) and reverse 5'-TACGAGCTGGTGGACTGAGA-3' (SEQ ID No. 17). β-tubulin was utilized as an internal control.

The PCR reaction mixture consists of cDNA derived from 50 ng of mRNA, 5 pmol of sense and antisense primers for both the TADG-15 gene and the β-tubulin gene, 200 µmol of dNTPs, 5 µCi of α-³²PdCTP and 0.25 units of Taq DNA polymerase with reaction buffer (Promega) in a final volume of 25 µl. The target sequences were amplified in parallel with the β-tubulin gene. Thirty cycles of PCR were carried out in a Thermal Cycler (Perkin Elmer Gene Amp 2400; Perkin-Elmer Cetus). Each cycle of PCR included 30 sec of denaturation at 94°C, 30 sec of annealing at 60°C and 30 sec of extension at 72°C. The annealing temperature varies according to the primers that are used in the PCR reaction. For the reactions involving degenerate primers, an annealing temperature of 48°C was used. The appropriate annealing temperature for the TADG-15- and β-tubulin-specific primers is 62°C.

A portion of the PCR products were separated on 2 % agarose gels and the radioactivity of each PCR product was determined by using a Phospholmager (Molecular Dynamics). In the present study, the expression ratio (TADG-15/β-tubulin) was used to evaluate gene expression and defined the value at mean ± 2SD of normal ovary as the cut-off value to determine overexpression. The student's *t* test was used for comparison of the mean values of normal ovary and tumors.

### EXAMPLE 7

### Immunohistochemistry

Immunohistochemical staining was performed using a Vectastain Elite ABC Kit (Vector). Formalin-fixed and paraffin-embedded specimens were routinely deparaffinized and processed using microwave heat treatment in 0.01 M sodium citrate buffer (pH 6.0). The specimens were incubated with normal goat serum in a moist chamber for 30 min. After incubation with biotinylated anti-rabbit IgG for 30 min, the sections were then incubated with ABC reagent (Vector) for 3 0 min. The final products were visualized using the AEC substrate system (DAKO) and sections were counterstained with hematoxylin before mounting. Negative controls were performed using normal serum instead of the primary antibody.

### EXAMPLE 8

### Antisense TADG-15

TADG-15 is cloned and expressed in the opposite orientation such that an antisense RNA molecule (SEQ ID No. 18) is produced. For example, the antisense RNA is used to hybridize to the complementary RNA in the cell and thereby inhibit translation of TADG-15 RNA into protein.

### EXAMPLE 9

### Peptide ranking

For vaccine or immune stimulation, individual 9-mers to 11-mers were examined to rank the binding of individual peptides to the top 8 haplotypes in the general population (Parker et al., (1994)). The computer program used for this analyses can be found at <http://www-bimas.dcrt.nih.gov/molbio/hla_bind/>. Table 1 shows the peptide ranking based upon the predicted half-life of each peptide's binding to a particular HLA allele. A larger half-life indicates a stronger association with that peptide and the particular HLA molecule. The TADG-15 peptides that strongly bind to an HLA allele are putative immunogens, and are used to innoculate an individual against TADG-15.

**TABLE 1**

| TADG-15 peptide ranking | | | | |
|---|---|---|---|---|
| HLA Type & Ranking | Start | Peptide | Predicted Dissociation_{1/2} | SEQ ID No. |
| HLA A0201 | | | | |
| 1 | 68 | VLLGIGFLV | 2537.396 | 19 |
| 2 | 126 | LLYSGVPFL | 1470.075 | 20 |
| 3 | 644 | SLISPNWLV | 521.640 | 21 |
| 4 | 379 | KVSFKFFYL | 396.525 | 22 |
| 5 | 386 | YLLEPGVPA | 346.677 | 23 |
| 6 | 257 | SLTFRSFDL | 123.902 | 24 |
| 7 | 762 | ILQKGEIRV | 118.238 | 25 |
| 8 | 841 | RLPLFRDWI | 106.842 | 26 |
| 9 | 64 | GLLLVLLGI | 88,783 | 27 |
| 10 | 57 | VLAAVLIGL | 83.527 | 28 |

| HLA A0205 | | | | |
|---|---|---|---|---|
| 1 | 67 | LVLLGIGFL | 142.800 | 29 |
| 2 | 379 | KVSFKFFYL | 100.800 | 30 |
| 3 | 126 | LLYSGVPFL | 71.400 | 31 |
| 4 | 88 | KVFNGYMRI | 36.000 | 32 |
| 5 | 670 | TQWTAFLGL | 33.600 | 33 |
| 6 | 119 | KVKDALKLL | 25.200 | 34 |
| 7 | 60 | AVLIGLLLV | 24.000 | 35 |
| 8 | 62 | LIGLLLVLL | 23.800 | 36 |
| 9 | 57 | VLAAVLIGL | 23.800 | 37 |
| 10 | 61 | VLIGLLLVL | 23.800 | 38 |

| HLA A1 | | | | |
|---|---|---|---|---|
| 1 | 146 | FSEGSVIAY | 337.500 | 39 |
| 2 | 658 | YIDDRGFRY | 125.000 | 40 |
| 3 | 449 | SSDPCPGQF | 75.000 | 41 |
| 4 | 401 | YVEINGEKY | 45.000 | 42 |
| 5 | 387 | LLEPGVPAG | 18.000 | 43 |
| 6 | 553 | GSDEASCPK | 15.000 | 44 |
| 7 | 97 | TNENFVDAY | 11.250 | 45 |
| 8 | 110 | STEFVSLAS | 11.250 | 46 |
| 9 | 811 | SVEADGRIF | 9.000 | 47 |
| 10 | 666 | YSDPTQWTA | 7.500 | 48 |

| HLA A24 | | | | |
|---|---|---|---|---|
| 1 | 709 | DYDIALLEL | 220.000 | 49 |
| 2 | 408 | KYCGERSQF | 200.000 | 50 |
| 3 | 754 | QYGGTGALI | 50.000 | 51 |
| 4 | 153 | AYYWSEFSI | 50.000 | 52 |
| 5 | 722 | EYSSMVRPI | 50.000 | 53 |
| 6 | 326 | GFEATFFQL | 36.000 | 54 |
| 7 | 304 | TFHSSQNVL | 24.000 | 55 |
| 8 | 707 | TFDYDIALL | 20.000 | 56 |
| 9 | 21 | KYNSRHEKV | 16.500 | 57 |
| 10 | 665 | RYSDPTQWT | 14.400 | 58 |

| HLA B7 | | | | |
|---|---|---|---|---|
| 1 | 686 | APGVQERRL | 240.000 | 59 |
| 2 | 12 | GPKDFGAGL | 80.000 | 60 |
| 3 | 668 | DPTQWTAFL | 80.000 | 61 |
| 4 | 461 | TGRCIRKEL | 60.000 | 62 |
| 5 | 59 | AAVLIGLLL | 36.000 | 63 |
| 6 | 379 | KVSFKFFYL | 20.000 | 64 |
| 7 | 119 | KVKDALKLL | 20.000 | 65 |
| 8 | 780 | LPQQITPRM | 20.000 | 66 |
| 9 | 67 | LVLLGIGFL | 20.000 | 67 |
| 10 | 283 | SPMEPHALV | 18.000 | 68 |

| HLA B8 | | | | |
|---|---|---|---|---|
| 1 | 12 | GPKDFGAGL | 24.000 | 69 |
| 2 | 257 | SLTFRSFDL | 8.000 | 70 |
| 3 | 180 | MLPPRARSL | 8.000 | 71 |
| 4 | 217 | GLHARGVEL | 8.000 | 72 |
| 5 | 173 | MAEERVVML | 4.800 | 73 |
| 6 | 267 | SCDERGSDL | 4.800 | 74 |
| 7 | 567 | CTKHTYRCL | 4.000 | 75 |
| 8 | 724 | SSMVRPICL | 4.000 | 76 |
| 9 | 409 | YCGERSQFV | 3.600 | 77 |
| 10 | 495 | TCKNKFCKP | 3.200 | 78 |

| HLA B2702 | | | | |
|---|---|---|---|---|
| 1 | 427 | VRFHSDQSY | 1000.000 | 79 |
| 2 | 695 | KRIISHPFF | 600.000 | 80 |
| 3 | 664 | FRYSDPTQW | 500.000 | 81 |
| 4 | 220 | ARGVELMRF | 200.000 | 82 |
| 5 | 492 | HQFTCKNKF | 100.000 | 83 |
| 6 | 53 | GRWVVLAAV | 100.000 | 84 |
| 7 | 248 | LRGDADSVL | 60.000 | 85 |
| 8 | 572 | YRCLNGLCL | 60.000 | 86 |
| 9 | 692 | RRLKRIISH | 60.000 | 87 |
| 10 | 24 | SRHEKVNGL | 60.000 | 88 |

| HLA B4403 | | | | |
|---|---|---|---|---|
| 1 | 147 | SEGSVIAYY | 360.000 | 89 |
| 2 | 715 | LELEKPAEY | 360.000 | 90 |
| 3 | 105 | YENSNSTEF | 60.000 | 91 |
| 4 | 14 | KDFGAGLKY | 50.625 | 92 |
| 5 | 129 | SGVPFLGPY | 36.000 | 93 |
| 6 | 436 | TDTGFLAEY | 33.750 | 94 |
| 7 | 766 | GEIRVINQT | 30.000 | 95 |
| 8 | 402 | VEINGEKYC | 30.000 | 96 |
| 9 | 482 | DELNCSCDA | 24.000 | 97 |
| 10 | 82 | RDVRVQKVF | 22.500 | 98 |

### EXAMPLE 10

### TADG-15 cDNA

A screening strategy to identify proteases which are overexpressed in human cancer has been developed in which RT-PCR products amplified specifically in tumors, as compared to normal tissue, are examined.⁹ During this effort, candidate genes were identified using redundant sense primers to the conserved amino acid histidine domain at the NH₃ end of the catalytic domain and antisense primers to the downstream conserved amino acid serine domain. Subcloning and sequencing the appropriate 480 base pair band(s) amplified in such a PCR reaction provides the basis for identifying the gene(s) encoding proteases(s). Among these amplified catalytic domains, a new serine protease gene named TADG-15 (tumor antigen-derived gene 15) was identified. The catalytic domain of the newly identified TADG-15 protein is similar to other serine proteases and specifically contains conserved amino acids appropriate for the catalytic domain of the trypsin-like serine protease family.

A computerized search of GenEMBL databases using the FASTA program (Wisconsin Package Version 9.1, GCG, Madison, Wisconsin) for amino acid sequences homologous to the TADG-15 protease domain revealed that homologies with other known human proteases never exceeds 55%. Figure 1 shows the alignment of the protease domain of TADG-15 compared with other human serine proteases. Using the BESTFIT program available through GCG, the similarities between TADG-15 and trypsin, chymotrypsin, and tissue-type plasminogen activator are 51%, 46% and 52%, respectively.

From the sequence derived from the TADG-15 catalytic domain, specific primers were synthesized to amplify a TADG-15-specific probe for library screening. After screening an ovarian carcinoma library, one 1785 bp clone was obtained which included the 3' end of the TADG-15 transcript. Upon further screening using the 5' end of the newly detected clone, two additional clones were identified which provided another 1362 bp of the cDNA, including the 5' end of the TADG-15 transcript. The total length of the sequenced cDNA was approximately 3.15 kb. The total nucleotide sequence obtained includes a Kozak's consensus sequence preceding a single open reading frame encoding a predicted protein of 855 amino acids (Figure 2).

The deduced open reading frame encoded by the TADG-15 nucleotide sequence (Figures 2, 3 and 4) contains several distinct domains as follows: an amino terminal cytoplasmic tail (amino acids (aa) #1-54), a potential transmembrane domain (aa #55-77), an extracellular membrane domain (aa #78-213), two complement subcomponents Clr/Cls, Uegf, and bone morphogenetic protein 1 (CUB) repeats (aa #214-447), four ligand binding repeats of the low density lipoprotein (LDL) receptor-like domain (aa #453-602) and a serine protease domain (aa #615-855). The TADG-15 protein also contains two potential N-linked glycosylation sites (aa #109 and 302) and a potential proteolytic cleavage site upstream from the protease domain (aa #614) which could release and/or activate the protease at the carboxy end of this protein. In addition, TADG-15 contains an RGD motif (aa #249-251) which is commonly found in proteins involved in cell-cell adhesion.

### EXAMPLE 11

### TADG-15 expression

To examine the size of the transcript for TADG-15 and its pattern of expression in various tissues, Northern blot hybridization was performed for representative histological types of carcinoma and in a series of cell lines, fetal tissues and normal adult tissues (Figure 5). The transcript size for the TADG-15 message was determined to be approximately 3.2 kb and a single intense transcript appeared to be present in all of the carcinomas examined, whereas no visible band was detected in normal ovary (Figure 5). This transcript size is also in good agreement with the sequence data predicting a transcript size of 3.15 kb. The ovarian tumor cell lines, SW626 and CAOV3, also showed an abundance of transcript, however little or no transcript was detectable in the breast carcinoma cell lines MDA-MB-231 and MDA-MB-4355. Among normal human fetal tissues, fetal kidney showed an abundance of the TADG-15 transcript and low expression was also detected in fetal lung. In normal adult tissues, TADG-15 was detected in colon with low levels of expression in small intestine and prostate (Figure 5).

To evaluate mRNA transcript expression of TADG-15 in ovarian tumors and normal ovary, semi-quantitative PCR (Figure 6) was performed. In a preliminary study, the linearity of this assay^{11,12} was confirmed and its efficacy correlated with both Northern blots and immunohistochemistry. The data was quantified using a phosphoimager and compared as a ratio of expression (TADG-15/β-tubulin). Results herein indicate that TADG-15 transcript expression is elevated above the cut-off value (mean for normal ovary ± 2 SD) in all of the tumor cases examined and is either not detected or detected at extremely low levels in normal ovaries (Figure 6A and B). Analysis of ovarian carcinoma subtypes, including early stage and late stage disease, confirms overexpression of TADG-15 in all carcinomas examined (Table 2). All of the carcinomas studied, which included 5 stage I and 3 stage II carcinomas, showed overexpression of the TADG-15 gene.

These data can also be examined with regard to tumor stage and histological sub-type, and results indicated that every carcinoma of every stage and histological sub-type overexpressed the TADG-15 gene. The expression ratio (mean value ± SD) for normal ovary group was determined as 0.182 ± 0.024, for LMP tumor group as 0.847 ± 0.419 and for carcinoma group as 0.771 ± 0.380 (Table 2). A comparison between the normal ovary group and tumor groups showed that overexpression of the TADG-15 gene is statistically significant in both the LMP tumor group and the carcinoma group (LMP tumor: p<0.001, carcinoma: p<0.0001).

As shown in Figure 6, TADG-15 transcripts were noted in all ovarian carcinomas, but were not present at detectable levels in any of the following tissues: a) normal ovary, b) fetal liver and brain, c) adult spleen, thymus, testes, ovary and peripheral blood lymphocytes, d) skeletal muscle, liver, brain or heart. This evaluation was extended to a standard panel of about 40 tumors. Using TADG-15-specific primers, the expression was also examined in tumor cell lines derived from both ovarian and breast carcinoma tissues as shown in Figure 7 and in other tumor tissues as shown in Figure 8. Expression of TADG-15 was also observed in carcinomas of the breast, colon, prostate and lung.

Polyclonal antibodies developed to a synthetic peptide (a 12-mer) at the carboxy terminus of the protease domain were used to examine TADG-15 expression in cell lines by Western blot and by immunolocalization in normal ovary and ovarian tumors. Western blots of cell extracts from SW626 and CAOV3 cells were probed with both antibody and preimmune sera (Figure 9). Several bands were detected with the antibody, including bands of approximately 100,000 daltons, approximately 60,000 daltons and 32,000 daltons. The anticipated molecular size of the complete TADG-15 molecule is estimated to be approximately 100,000 daltons, and the protease domain which may be released by proteolytic cleavage at aa #614 is estimated to be approximately 32,000 daltons. Some intermediate proteolytic product may be represented by the 60,000 dalton band.

Antibody staining of tumor cells confirms the presence of the TADG-15 protease in the cytoplasm of a serous LMP tumor, mucinous LMP tumor and serous carcinoma (Figure 10B, C & D, respectively). This diffuse staining pattern may be due to detection of TADG15 within the cell as it is being packaged and transported to the cell surface. In endometrioid carcinoma, the antigen is clearly detectable on the surface of tumor cells (Figure 10E). No staining was detected in normal ovarian epithelium or stromal cells (Figure 10A). Immunohistochemical staining of a series of 27 tumors indicates the presence of the TADG-15 protein in all the carcinoma subtypes examined, including the low malignant potential group. Strong staining was noted in 7 of 9 low malignant potential tumors and 13 of 18 carcinomas (Table 3).

**TABLE 2**

| Number of cases with overexpression of TADG-15 in normal ovaries and ovarian tumors | | | |
|---|---|---|---|
| | N | overexpression of TADG-15 | expression ratio^{a} |
| Normal | 10 | 0 (0%) | 0.182 ± 0.024 |
| LMP | 10 | 10 (100%) | 0.847 ± 0.419 |
| serous | 6 | 6 (100%) | 0.862 ± 0.419 |
| mucinous | 4 | 4 (100%) | 0.825 ± 0.483 |
| Carcinoma | 31 | 31 (100%) | 0.771 ± 0.380 |
| serous | 18 | 18 (100%) | 0.779 ± 0.332 |
| mucinous | 7 | 7 (100%) | 0.907 ± 0.584 |
| endometrioid | 3 | 3 (100%) | 0.502 ± 0.083 |
| clear cell | 3 | 3 (100%) | 0.672. ± 0.077 |

| | | | |
|---|---|---|---|
| ^{a}The ratio of expression level of TADG-15 to β-tubulin (mean ± SD) | | | |

**TABLE 3**

| Immunohistochemical staining using TADG-15 | | |
|---|---|---|
| Lab No. | Histology | TADG-15 |
| | Surface epithelium of the ovary | - |
| H-3194 | serous (LMP) | ++ |
| H-162 | serous (LMP) | ++ |
| H-1182 | serous (LMP) | ++ |
| H-4818 | serous (LMP) | ++ |
| H-4881 | serous (LMP) | ++ |
| H-675 | mucinous (LMP) | + |
| H-2446 | mucinous (LMP) | + |
| H-0707 | mucinous (LMP) | ++ |
| H-2042 | mucinous (LMP) | ++ |
| H-2555 | serous carcinoma | ++ |
| H-1858 | serous carcinoma | ++ |
| H-5266 | serous carcinoma | ++ |
| H-5316 | serous carcinoma | + |
| H-2597 | serous carcinoma | + |
| H-4931 | mucinous carcinoma | ++ |
| H-1867 | mucinous carcinoma | ++ |
| H-5998 | mucinous carcinoma | ++ |
| H-2679 | endometrioid adenocarcinoma | + |
| H-5718 | endometrioid adenocarcinoma | ++ |
| H-3993 | endometrioid adenocarcinoma | + |
| H-2991 | endometrioid adenocarcinoma | ++ |
| H-2489 | endometrioid adenocarcinoma | ++ |
| H-5994 | clear cell carcinoma | ++ |
| H-6718 | clear cell carcinoma | ++ |
| H-1661 | clear cell carcinoma | ++ |
| H-6201 | clear cell carcinoma | ++ |
| H-5640 | clear cellr carcinoma | + |

| | | |
|---|---|---|
| - Negative; + Weak Positive; ++ Strong Positive (more than 50% of cell staining) | | |

### EXAMPLE 12

### TADG-15 homology

Recently, a mouse protein named epithin (GenBank Accession No. AF042822) has been described.¹⁴ Epithin is a 902 amino acid protein which contains a similar structure to TADG-15 in that it has a cytoplasmic domain, transmembrane domain, two CUB domains, four LDLR-like domains and a carboxy terminal serine protease domain. TADG-15 and epithin are 84% similar over 843 amino acids, suggesting that the proteins may be orthologous (Figure 11). The precise role of epithin remains to be elucidated.

A search of GeneBank for similar previously identified sequences yielded one such sequence with relatively high homology to a portion of the TADG-15 gene. The similarity between the portion of TADG-15 from nucleotide #182 to 3139 and SNC-19 GeneBank Accession No. #U20428) is approximately 97% (Figure 12). There are however significant differences between SNC-19 and TADG-15. For example, TADG-15 has an open reading frame of 855 amino acids whereas the longest open reading frame of SNC-19 is 173 amino acids. Additionally, SNC-19 does not include a proper start site for the initiation of translation, nor does it include the amino terminal portion of the protein encoded by TADG-15. Moreover, SNC-19 does not include an open reading frame for a functional serine protease because the His, Asp and Ser residues of the catalytic triad that are necessary for function are encoded in different reading frames.

### Implications

The overall structure of the TADG-15 protein is relatively similar to the members of the tolloid/BMP-1 family and the complement subcomponents, Clr/Cls. These proteins contain both CUB and protease domains, and complex formation through the ligand binding domain is essential for their function. Activation of the serine protease domains of Clr and Cls requires proteolytic cleavage of Arg-Gly and Arg-Ile bonds, respectively.¹⁵ Similarly, it might be expected that the TADG-15 protein is synthesized as a zymogen, which is activated by cleavage between Arg⁶¹⁴ and Val⁶¹⁵ and analogous to the activation mechanism of other serine protease zymogens. Western blot analysis of cultured cell lysates confirmed both a 100 kDa and 32 kDa peptide, which correspond to the putative zymogen (whole molecule) and a cleaved protease product of TADG-15 (Figure 9). These data support a model for proteolytic release and/or activation of TADG-15 as occurs for similar type II serine proteases.

CUB domains were first found in complement subcomponents C1r/C1s¹⁶⁻¹⁸ and are known to be a widespread module in developmentally regulated proteins, such as the bone morphogenetic protein-1 (BMP-1) and the tolloid gene product.¹⁸⁻²⁰ The role of these repeats remains largely unknown. However, some models suggest that the CUB domain may be involved in protein-protein interactions. The CUB domain of Clr and Cls participates in the assembly of the Cls-Clr-Clr-Cls tetrameric complex in the activation of the classical pathway of complement by providing protein-protein interaction domains.¹⁵ The *Drosophila* decapentaplegic (DPP) protein is essential for dorsal-ventral specification of the embryo, and the *Drosophila* tolloid (TLD) forms a complex with DPP to regulate its activity.^{19,20} Missense mutations in the CUB domain of the tolloid protein results in a phenotype that does not allow a protein interaction with the DPP complex.¹⁹

The TADG-15 protein contains two tandem repeats of CUB-like domains between amino acid residues 214 and 447. Each of these is approximately 110 amino acids long and each has four conserved cysteine residues characteristic of other CUBs (amino acids 214, 244, 268, 294, 340, 366, 397, 410). By analogy, the CUB repeats of the TADG-15 protein may form an interactive domain capable of promoting multimeric complex formation and regulating the activity of the target protein or TADG-15 itself.

The TADG-15 protein also contains the LDL receptor ligand binding repeat (class A motif) -like domain, which consists of four contiguous cysteine-rich repeats (amino acid residues 453 to 602). Each cysteine-rich repeat is approximately 40 amino acids long and contains a conserved, negatively-charged sequence (Ser-Asp-Glu) with six cysteine residues. In the LDL receptor protein, this repeat is thought to function as a protein-binding domain which interacts with the lysine and arginine residues present in lipoproteins.^{21,22} In addition, the first repeat of the LDL receptor appears to bind Ca²⁺ and not the lipoproteins.²³ By analogy, it is possible that the LDL receptor-like repeat in TADG-15 may act in a similar fashion, interacting with positively charged regions of other proteins and/or as a Ca²⁺ binding site. As a result of ligand binding and the formation of receptor-ligand complex, LDL receptor is internalized via clathrin-coated pits.²⁴ These types of plasma membrane receptors contain a characteristic amino acid sequence in their cytoplasmic domain for binding to clathrin-coated pits.²⁴ TADG-15 does not contain this motif in its cytosolic region, and furthermore, no similarities with other known protein sequences were found in the cytoplasmic domain of the TADG-15. This finding suggests that TADG-15 functions in a different manner from the endocytic receptors (such as the LDL receptor), although TADG-15 possesses similar ligand-binding repeats in the extracellular matrix.

Although the precise role of TADG-15 is unknown, this gene is clearly overexpressed in ovarian tumors. A variety of proteases, such as type IV collagenase and plasminogen activator, appear to be involved in the process of tumor invasion and are constituents of a protease cascade in malignant progression. TADG-15 may constitute such an activity and directly digest extracellular matrix components surrounding a tumor, or activate other proteases by cleavage of inactive precursors, indirectly enhancing tumor growth and invasion. It is also possible that TADG-15 may function like a member of the tolloid/BMP-1 family by forming complexes with other growth factors or signal transduction proteins to modulate their activities.

These data raise the possibility that the TADG-15 gene and its translated protein will be a useful marker for the early detection of ovarian carcinoma through release of the protease domain into the extracellular matrix and ultimately the circulation. These data also suggest the possibility of using TADG-15 as a target for therapeutic intervention through delivery systems directed at the CUB/LDLR ligand binding domains.

The following references were cited herein:
1. Liotta, L.A., et al. Cell, 64: 327-336, 1991.
2. Duffy, M.J. Clin. Exp. Metastasis, 10: 145-155, 1992.
3. Tryggvason, K., et al. Biochem. Biophys. Acta., 907: 191-217, 1987.
4. Levy, A.T., et al. Cancer Res., 51: 439-444, 1991.
5. Monsky, W.L. et al. Cancer Res., 53: 3159-3164, 1993.
6. Duffy, M.J. et al. Cancer, 62: 531-533, 1988.
7. Häckel, C., et al. Cancer, 79: 53-58, 1997.
8. Watt, et al. Proc.Natl. Acad. Sci.U.S.A., 83:3166-3170, 1986.
9. Tanimoto, H. et al. Cancer Res., 57: 2884-2887, 1997.
11. Shigemasa, K. et al. J. Soc. Gynecol. Invest., 4: 95-102, 1997.
12. Tanimoto, H. et al. Gynecol. Oncol., 66: 308-312, 1997.
13. Maniatis, T., Fritsch, E.F. & Sambrook, J. Molecular Cloning, p. 309-361 Cold Spring Harbor Laboratory, New York, 1982.
14. Kim, M.G., et al. Immunogenetics, 49(5): 420-428, 1999.
15. Arlaud et al. Method in Enzymology, 223: 61-82, 1993.
16. Journet, A. & Tosi, M. Biochem. J., 240: 783-787, 1986.
17. Mackinnon, et al. Eur. J. Biochem., 169: 547-553, 1987.
18. Bork, P. & Beckmann, G. J. Mol. Biol., 231: 539-545, 1993.
19. Childs, & O'Connor, M.B. Dev. Biol., 162: 209-220, 1994.
20. Blader, PL, et al. Science, 278: 1937-1940, 1997.
21. Yamamoto, T. et al. Cell, 39: 27-38, 1984.
22. Daly, N.L., et al. Proc. Natl. Acad. Sci., 92: 6334-6338, 1995.
23. van Driel, I.R., et al. J. Biol. Chem., 262: 17443-17449, 1987.
24. Lodish, H. et al. Sorting of membrane proteins internalized from the cell surface. In: Molecular Cell Biology, 3rd ed., p.722-733 Scientific American Books, Inc., New York, 1995.
25. Parker, KC et al. J. Immunol. 152:163, 1994.

Any patents or publications mentioned in this specification are indicative of the levels of those skilled in the art to which the invention pertains. These patents and publications are herein incorporated by reference to the same extent as if each individual publication was specifically and individually indicated to be incorporated by reference.

One skilled in the art will readily appreciate that the present invention is well adapted to carry out the objects and obtain the ends and advantages mentioned, as well as those inherent therein. The present examples along with the methods, procedures, treatments, molecules, and specific compounds described herein are presently representative of preferred embodiments, are exemplary, and are not intended as limitations on the scope of the invention. Changes therein and other uses will occur to those skilled in the art which are encompassed within the spirit of the invention as defined by the scope of the claims.

## Claims

1. An immunogenic composition, comprising an immunogenic fragment of a TADG-15 protein and an appropriate adjuvant.

2. The immunogenic composition of claim 1, wherein said fragment has 9-20 residues.

3. The immunogenic composition of claim 2, wherein said 9-residue fragment has the sequence shown in SEQ ID NOS: 2, 19, 20, 21, 29, 39,49, 50, 59, 79, 80, 81, 82, 83, 84, 89 and 90.

4. Use of the immunogenic composition of claims 1 to 3 in the production of a vaccine to inoculate an individual against TADG-15, wherein inoculation with said immunogenic composition elicits an immune response in the individual.

5. Use of the immunogenic composition of claims 1 to 3 to produce immune-activated cells directed toward TADG-15, wherein exposure to the immunogenic composition activates the dendritic cells against TADG-15.

6. Use according to claim 5, wherein the immune-activated cells are B-cells, T-cells or dendrites.

7. Use according to claims 5 or 6, wherein the dendritic cells are isolated from an individual prior to exposure, said activated dendritic cells are reintroduced into said individual subsequent to said exposure.

8. Use according to any of claims 5 to 7, wherein said individual has cancer, is suspected of having cancer or is at risk of getting cancer.


Sequence Listing

<110> The Board of Trustees of the University of Arkansas

<120> TADG-15: An Extracellular Serine Protease
Overexpressed in Carcinomas

<130> APM/JM/8695 EP

<140> EP 00973739.6
<141> 2000-10-20

<150> PCT/US00/29095
<151> 2000-10-20

<150> US 09/421,213
<151> 1999-10-20

<160> 98

<210> 1
<211> 3147
<212> DNA
<213> Homo sapiens

<220>
<223> TADG-15

<400> 1
tcaagagcgg cctcggggta ccatggggag cgatcgggcc cgcaagggcg 50
gagggggccc gaaggacttc ggcgcgggac tcaagtacaa ctcccggcac 100
gagaaagtga atggcttgga ggaaggcgtg gagttcctgc cagtcaacaa 150
cgtcaagaag gtggaaaagc atggcccggg gcgctgggtg gtgctggcag 200
ccgtgctgat cggcctcctc ttggtcttgc tggggatcgg cttcctggtg 250
tggcatttgc agtaccggga cgtgcgtgtc cagaaggtct tcaatggcta 300
catgaggatc acaaatgaga attttgtgga tgcctacgag aactccaact 350
ccactgagtt tgtaagcctg gccagcaagg tgaaggacgc gctgaagctg 400
ctgtacagcg gagtcccatt cctgggcccc taccacaagg agtcggctgt 450

gacggccttc agcgagggca gcgtcatcgc ctactactgg tctgagttca 500
gcatcccgca gcacctggtg gaggaggccg agcgcgtcat ggccgaggag 550
cgcgtagtca tgctgccccc gcgggcgcgc tccctgaagt cctttgtggt 600
cacctcagtg gtggctttcc ccacggactc caaaacagta cagaggaccc 650
aggacaacag ctgcagcttt ggcctgcacg cccgcggtgt ggagctgatg 700
cgcttcacca cgcccggctt ccctgacagc ccctaccccg ctcatgcccg 750
ctgccagtgg gccctgcggg gggacgccga ctcagtgctg agcctcacct 800
tccgcagctt tgaccttgcg tcctgcgacg agcgcggcag cgacctggtg 850
acggtgtaca acaccctgag ccccatggag ccccacgccc tggtgcagtt 900
gtgtggcacc taccctccct cctacaacct gaccttccac tcctcccaga 950
acgtcctgct catcacactg ataaccaaca ctgagcggcg gcatcccggc 1000
tttgaggcca ccttcttcca gctgcctagg atgagcagct gtggaggccg 1050
cttacgtaaa gcccagggga cattcaacag cccctactac ccaggccact 1100
acccacccaa cattgactgc acatggaaca ttgaggtgcc caacaaccag 1150
catgtgaagg tgagcttcaa attcttctac ctgctggagc ccggcgtgcc 1200
tgcgggcacc tgccccaagg actacgtgga gatcaatggg gagaaatact 1250
gcggagagag gtcccagttc gtcgtcacca gcaacagcaa caagatcaca 1300
gttcgcttcc actcagatca gtcctacacc gacaccggct tcttagctga 1350
atacctctcc tacgactcca gtgacccatg cccggggcag ttcacgtgcc 1400
gcacggggcg gtgtatccgg aaggagctgc gctgtgatgg ctgggccgac 1450
tgcaccgacc acagcgatga gctcaactgc agttgcgacg ccggccacca 1500
gttcacgtgc aagaacaagt tctgcaagcc cctcttctgg gtctgcgaca 1550
gtgtgaacga ctgcggagac aacagcgacg agcaggggtg cagttgtccg 1600
gcccagacct tcaggtgttc caatgggaag tgcctctcga aaagccagca 1650
gtgcaatggg aaggacgact gtggggacgg gtccgacgag gcctcctgcc 1700
ccaaggtgaa cgtcgtcact tgtaccaaac acacctaccg ctgcctcaat 1750
gggctctgct tgagcaaggg caaccctgag tgtgacggga aggaggactg 1800
tagcgacggc tcagatgaga aggactgcga ctgtgggctg cggtcattca 1850
cgagacaggc tcgtgttgtt gggggcacgg atgcggatga gggcgagtgg 1900
ccctggcagg taagcctgca tgctctgggc cagggccaca tctgcggtgc 1950
ttccctcatc tctcccaact ggctggtctc tgccgcacac tgctacatcg 2000
atgacagagg attcaggtac tcagacccca cgcagtggac ggccttcctg 2050
ggcttgcacg accagagcca gcgcagcgcc cctggggtgc aggagcgcag 2100
gctcaagcgc atcatctccc accccttctt caatgacttc accttcgact 2150
atgacatcgc gctgctggag ctggagaaac cggcagagta cagctccatg 2200
gtgcggccca tctgcctgcc ggacgcctcc catgtcttcc ctgccggcaa 2250
ggccatctgg gtcacgggct ggggacacac ccagtatgga ggcactggcg 2300
cgctgatcct gcaaaagggt gagatccgcg tcatcaacca gaccacctgc 2350

gagaacctcc tgccgcagca gatcacgccg cgcatgatgt gcgtgggctt 2400
cctcagcggc ggcgtggact cctgccaggg tgattccggg ggacccctgt 2450
ccagcgtgga ggcggatggg cggatcttcc aggccggtgt ggtgagctgg 2500
ggagacggct gcgctcagag gaacaagcca ggcgtgtaca caaggctccc 2550
tctgtttcgg gactggatca aagagaacac tggggtatag ggccggggcc 2600
cacccaaatg tgtacacctg cggggccacc catcgtccac cccagtgtgc 2650
acgcctgcag gctggagact ggaccgctga ctgcaccagc gcccccagaa 2700
catacactgt gaactcaatc tccagggctc caaatctgcc tagaaaacct 2750
ctcgcttcct cagcctccaa agtggagctg ggaggtagaa ggggaggaca 2800
ctggtggttc tactgaccca actgggggca aaggtttgaa gacacagcct 2850
cccccgccag ccccaagctg ggccgaggcg cgtttgtgta tatctgcctc 2900
ccctgtctgt aaggagcagc gggaacggag cttcggagcc tcctcagtga 2950
aggtggtggg gctgccggat ctgggctgtg gggcccttgg gccacgctct 3000
tgaggaagcc caggctcgga ggaccctgga aaacagacgg gtctgagact 3050
gaaattgttt taccagctcc cagggtggac ttcagtgtgt gtatttgtgt 3100
aaatgggtaa aacaatttat ttctttttaa aaaaaaaaaa aaaaaaa 3147

<210> 2
<211> 855
<212> PRT
<213> Homo sapiens

<220>
<223> TADG-15

<400> 2

<210> 3
<211> 256
<212> PRT
<213> Homo sapiens

<220>
<223> Hepsin

<400> 3
Arg Ile Val Gly Gly Arg Asp Thr Ser Leu Gly Arg Trp Pro Trp
                5                   10                  15

Gln Val Ser Leu Arg Tyr Asp Gly Ala His Leu Cys Gly Gly Ser
                20                  25                  30
Leu Leu Ser Gly Asp Trp Val Leu Thr Ala Ala His Cys Phe Pro
                35                  40                  45
Glu Arg Asn Arg Val Leu Ser Arg Trp Arg Val Phe Ala Gly Ala
                50                  55                  60
Val Ala Gln Ala Ser Pro His Gly Leu Gln Leu Gly Val Gln Ala
                65                  70                  75
Val Val Tyr His Gly Gly Tyr Leu Pro Phe Arg Asp Pro Asn Ser
                80                  85                  90
Glu Glu Asn Ser Asn Asp Ile Ala Leu Val His Leu Ser Ser Pro
                95                  100                 105
Leu Pro Leu Thr Glu Tyr Ile Gln Pro Val Cys Leu Pro Ala Ala
                110                 115                 120
Gly Gln Ala Leu Val Asp Gly Lys Ile Cys Thr Val Thr Gly Trp
                125                 130                 135
Gly Asn Thr Gln Tyr Tyr Gly Gln Gln Ala Gly Val Leu Gln Glu
                140                 145                 150
Ala Arg Val Pro Ile Ile Ser Asn Asp Val Cys Asn Gly Ala Asp
                155                 160                 165
Phe Tyr Gly Asn Gln Ile Lys Pro Lys Met Phe Cys Ala Gly Tyr
                170                 175                 180
Pro Glu Gly Gly Ile Asp Ala Cys Gln Gly Asp Ser Gly Gly Pro
                185                 190                 195
Phe Val Cys Glu Asp Ser Ile Ser Arg Thr Pro Arg Trp Arg Leu
                200                 205                 210
Cys Gly Ile Val Ser Trp Gly Thr Gly Cys Ala Leu Ala Gln Lys
                215                 220                 225
Pro Gly Val Tyr Thr Lys Val Ser Asp Phe Arg Glu Trp Ile Phe
                230                 235                 240
Gln Ala Ile Lys Thr His Ser Glu Ala Ser Gly Met Val Thr Gln
                245                 250                 255
Leu

<210>  4

<211>  225

<212>  PRT

<213>  Homo sapiens

<220>

<223>  SCCE

<400>  4

Lys Ile Ile Asp Gly Ala Pro Cys Ala Arg Gly Ser His Pro Trp
                5                   10                  15

Gln Val Ala Leu Leu Ser Gly Asn Gln Leu His Cys Gly Gly Val
                20                  25                  30

Leu Val Asn Glu Arg Trp Val Leu Thr Ala Ala His Cys Lys Met
                35                  40                  45

Asn Glu Tyr Thr Val His Leu Gly Ser Asp Thr Leu Gly Asp Arg
                50                  55                  60

Arg Ala Gln Arg Ile Lys Ala Ser Lys Ser Phe Arg His Pro Gly
                65                  70                  75

Tyr Ser Thr Gln Thr His Val Asn Asp Leu Met Leu Val Lys Leu
                80                  85                  90

Asn Ser Gln Ala Arg Leu Ser Ser Met Val Lys Lys Val Arg Leu
                95                  100                 105

Pro Ser Arg Cys Glu Pro Pro Gly Thr Thr Cys Thr Val Ser Gly
                110                 115                 120

Trp Gly Thr Thr Thr Ser Pro Asp Val Thr Phe Pro Ser Asp Leu
                125                 130                 135

Met Cys Val Asp Val Lys Leu Ile Ser Pro Gln Asp Cys Thr Lys
                140                 145                 150

Val Tyr Lys Asp Leu Leu Glu Asn Ser Met Leu Cys Ala Gly Ile
                155                 160                 165

Pro Asp Ser Lys Lys Asn Ala Cys Asn Gly Asp Ser Gly Gly Pro
                170                 175                 180

Leu Val Cys Arg Gly Thr Leu Gln Gly Leu Val Ser Trp Gly Thr
                185                 190                 195

Phe Pro Cys Gly Gln Pro Asn Asp Pro Gly Val Tyr Thr Gln Val
                200                 205                 210

Cys Lys Phe Thr Lys Trp Ile Asn Asp Thr Met Lys Lys His Arg
                215                 220                 225

<210> 5
<211> 225
<212> PRT
<213> Homo sapiens

<220>
<223> Trypsin

<400> 5
Lys Ile Val Gly Gly Tyr Asn Cys Glu Glu Asn Ser Val Pro Tyr
                5                   10                  15
Gln Val Ser Leu Asn Ser Gly Tyr His Phe Cys Gly Gly Ser Leu
                20                  25                  30
Ile Asn Glu Gln Trp Val Val Ser Ala Gly His Cys Tyr Lys Ser
                35                  40                  45
Arg Ile Gln Val Arg Leu Gly Glu His Asn Ile Glu Val Leu Glu
                50                  55                  60
Gly Asn Glu Gln Phe Ile Asn Ala Ala Lys Ile Ile Arg His Pro
                65                  70                  75
Gln Tyr Asp Arg Lys Thr Leu Asn Asn Asp Ile Met Leu Ile Lys
                80                  85                  90
Leu Ser Ser Arg Ala Val Ile Asn Ala Arg Val Ser Thr Ile Ser
                95                  100                 105
Leu Pro Thr Ala Pro Pro Ala Thr Gly Thr Lys Cys Leu Ile Ser
                110                 115                 120
Gly Trp Gly Asn Thr Ala Ser Ser Gly Ala Asp Tyr Pro Asp Glu
                125                 130                 135
Leu Gln Cys Leu Asp Ala Pro Val Leu Ser Gln Ala Lys Cys Glu
                140                 145                 150
Ala Ser Tyr Pro Gly Lys Ile Thr Ser Asn Met Phe Cys Val Gly
                155                 160                 165
Phe Leu Glu Gly Gly Lys Asp Ser Cys Gln Gly Asp Ser Gly Gly
                170                 175                 180
Pro Val Val Cys Asn Gly Gln Leu Gln Gly Val Val Ser Trp Gly
                185                 190                 195

Asp Gly Cys Ala Gln Lys Asn Lys Pro Gly Val Tyr Thr Lys Val
                200                 205                 210
Tyr Asn Tyr Val Lys Trp Ile Lys Asn Thr Ile Ala Ala Asn Ser
                215                 220                 225

<210> 6
<211> 231
<212> PRT
<213> Homo sapiens

<220>
<223> Chymotrypsin

<400> 6
Arg Ile Val Asn Gly Glu Asp Ala Val Pro Gly Ser Trp Pro Trp
                5                   10                  15
Gln Val Ser Leu Gln Asp Lys Thr Gly Phe His Phe Cys Gly Gly
                20                  25                  30
Ser Leu Ile Ser Glu Asp Trp Val Val Thr Ala Ala His Cys Gly
                35                  40                  45
Val Arg Thr Ser Asp Val Val Val Ala Gly Glu Phe Asp Gln Gly
                50                  55                  60
Ser Asp Glu Glu Asn Ile Gln Val Leu Lys Ile Ala Lys Val Phe
                65                  70                  75
Lys Asn Pro Lys Phe Ser Ile Leu Thr Val Asn Asn Asp Ile Thr
                80                  85                  90
Leu Leu Lys Leu Ala Thr Pro Ala Arg Phe Ser Gln Thr Val Ser
                95                  100                 105
Ala Val Cys Leu Pro Ser Ala Asp Asp Asp Phe Pro Ala Gly Thr
                110                 115                 120
Leu Cys Ala Thr Thr Gly Trp Gly Lys Thr Lys Tyr Asn Ala Asn
                125                 130                 135
Lys Thr Pro Asp Lys Leu Gln Gln Ala Ala Leu Pro Leu Leu Ser
                140                 145                 150
Asn Ala Glu Cys Lys Lys Ser Trp Gly Arg Arg Ile Thr Asp Val
                155                 160                 165
Met Ile Cys Ala Gly Ala Ser Gly Val Ser Ser Cys Met Gly Asp
                170                 175                 180

Ser Gly Gly Pro Leu Val Cys Gln Lys Asp Gly Ala Trp Thr Leu
                185                 190                 195
Val Gly Ile Val Ser Trp Gly Ser Asp Thr Cys Ser Thr Ser Ser
                200                 205                 210
Pro Gly Val Tyr Ala Arg Val Thr Lys Leu Ile Pro Trp Val Gln
                215                 220                 225
Lys Ile Leu Ala Ala Asn
                230

<210>  7
<211>  255
<212>  PRT
<213>  Homo sapiens

<220>
<223>  Factor 7

<400>  7
Arg Ile Val Gly Gly Lys Val Cys Pro Lys Gly Glu Cys Pro Trp
                5                   10                  15
Gln Val Leu Leu Leu Val Asn Gly Ala Gln Leu Cys Gly Gly Thr
                20                  25                  30
Leu Ile Asn Thr Ile Trp Val Val Ser Ala Ala His Cys Phe Asp
                35                  40                  45
Lys Ile Lys Asn Trp Arg Asn Leu Ile Ala Val Leu Gly Glu His
                50                  55                  60
Asp Leu Ser Glu His Asp Gly Asp Glu Gln Ser Arg Arg Val Ala
                65                  70                  75
Gln Val Ile Ile Pro Ser Thr Tyr Val Pro Gly Thr Thr Asn His
                80                  85                  90
Asp Ile Ala Leu Leu Arg Leu His Gln Pro Val Val Leu Thr Asp
                95                  100                 105
His Val Val Pro Leu Cys Leu Pro Glu Arg Thr Phe Ser Glu Arg
                110                 115                 120
Thr Leu Ala Phe Val Arg Phe Ser Leu Val Ser Gly Trp Gly Gln
                125                 130                 135
Leu Leu Asp Arg Gly Ala Thr Ala Leu Glu Leu Met Val Leu Asn
                140                 145                 150

Val Pro Arg Leu Met Thr Gln Asp Cys Leu Gln Gln Ser Arg Lys
                155                 160                 165
Val Gly Asp Ser Pro Asn Ile Thr Glu Tyr Met Phe Cys Ala Gly
                170                 175                 180
Tyr Ser Asp Gly Ser Lys Asp Ser Cys Lys Gly Asp Ser Gly Gly
                185                 190                 195
Pro His Ala Thr His Tyr Arg Gly Thr Trp Tyr Leu Thr Gly Ile
                200                 205                 210
Val Ser Trp Gly Gln Gly Cys Ala Thr Val Gly His Phe Gly Val
                215                 220                 225
Tyr Thr Arg Val Ser Gln Tyr Ile Glu Trp Leu Gln Lys Leu Met
                230                 235                 240
Arg Ser Glu Pro Arg Pro Gly Val Leu Leu Arg Ala Pro Phe Pro
                245                 250                 255

<210>  8
<211>  253
<212>  PRT
<213>  Homo sapiens

<220>
<223>  Tissue plasminogen activator

<400>  8
Arg Ile Lys Gly Gly Leu Phe Ala Asp Ile Ala Ser His Pro Trp
                5                   10                  15
Gln Ala Ala Ile Phe Ala Lys His Arg Arg Ser Pro Gly Glu Arg
                20                  25                  30
Phe Leu Cys Gly Gly Ile Leu Ile Ser Ser Cys Trp Ile Leu Ser
                35                  40                  45
Ala Ala His Cys Phe Gln Glu Arg Phe Pro Pro His His Leu Thr
                50                  55                  60
Val Ile Leu Gly Arg Thr Tyr Arg Val Val Pro Gly Glu Glu Glu
                65                  70                  75
Gln Lys Phe Glu Val Glu Lys Tyr Ile Val His Lys Glu Phe Asp
                80                  85                  90
Asp Asp Thr Tyr Asp Asn Asp Ile Ala Leu Leu Gln Leu Lys Ser
                95                  100                 105

Asp Ser Ser Arg Cys Ala Gln Glu Ser Ser Val Val Arg Thr Val
                110                 115                 120
Cys Leu Pro Pro Ala Asp Leu Gln Leu Pro Asp Trp Thr Glu Cys
                125                 130                 135
Glu Leu Ser Gly Tyr Gly Lys His Glu Ala Leu Ser Pro Phe Tyr
                140                 145                 150
Ser Glu Arg Leu Lys Glu Ala His Val Arg Leu Tyr Pro Ser Ser
                155                 160                 165
Arg Cys Thr Ser Gln His Leu Leu Asn Arg Thr Val Thr Asp Asn
                170                 175                 180
Met Leu Cys Ala Gly Asp Thr Arg Ser Gly Gly Pro Gln Ala Asn
                185                 190                 195
Leu His Asp Ala Cys Gln Gly Asp Ser Gly Gly Pro Leu Val Cys
                200                 205                 210
Leu Asn Asp Gly Arg Met Thr Leu Val Gly Ile Ile Ser Trp Gly
                215                 220                 225
Leu Gly Cys Gly Gln Lys Asp Val Pro Gly Val Tyr Thr Lys Val
                230                 235                 240
Thr Asn Tyr Leu Asp Trp Ile Arg Asp Asn Met Arg Pro
                245                 250

<210> 9
<211> 2900
<212> DNA
<213> Homo sapiens

<220>
<223> SNC-19; GeneBank Accession No. #U20428

<400> 9
cgctgggtgg tgctggcagc cgtgctgatc ggcctcctct tggtcttgct 50
ggggatcggc ttcctggtgt ggcatttgca gtaccgggac gtgcgtgtcc 100
agaaggtctt caatggctac atgaggatca caaatgagaa ttttgtggat 150
gcctacgaga actccaactc cactgagttt gtaagcctgg ccagcaaggt 200
gaaggacgcg ctgaagctgc tgtacagcgg agtcccattc ctgggcccct 250
accacaagga gtcggctgtg acggccttca gcgagggcag cgtcatcgcc 300
tactactggt ctgagttcag catcccgcag cacctggttg aggaggccga 350
gcgcgtcatg gccaggagcg cgtagtcatg ctgcccccgc gggcgcgctc 400

cctgaagtcc tttgtggtca cctcagtggt ggctttcccc acggactcca 450
aaacagtaca gaggacccag gacaacagct gcagctttgg cctgcacgcc 500
gcggtgtgga gctgatgcgc ttcaccacgc cggcttccct gacagcccct 550
accccgctca tgcccgctgc cagtgggctg cggggacgcg acgcagtgct 600
gagctactcg agctgactcg cagcttgact gcgcctcgac gagcgcggca 650
gcgacctggt gacgtgtaca acaccctgag ccccatggag ccccacgcct 700
ggtgagtgtg tggcacctac cctccctcct acaacctgac cttccactcc 750
ctcccacgaa cgtcctgctc atcacactga taaccaacac tgacgcggca 800
tcccggcttt gaggccacct tcttccagct gcctaggatg agcagctgtg 850
gaggccgctt acgtaaagcc caggggacat tcaacagccc ctactaccca 900
ggccactacc cacccaacat tgactgcaca tggaaaattg aggtgcccaa 950
caaccagcat gtgaaggtgc gcttcaaatt cttctacctg ctggagcccg 1000
gcgtgcctgc gggcacctgc cccaaggact acgtggagat caatggggag 1050
aaatactgcg gagagaggtc ccagttcgtc gtcaccagca acagcaacaa 1100
gatcacagtt cgcttccact cagatcagtc ctacaccgac accggcttct 1150
tagctgaata cctctcctac gactccagtg acccatgccc ggggcagttc 1200
acgtgccgca cggggcggtg tatccggaag gagctgcgct gtgatggctg 1250
ggcgactgca ccgaccacag cgatgagctc aactgcagtt gcgacgccgg 1300
ccaccagttc acgtgcaaga gcaagttctg caagctcttc tgggtctgcg 1350
acagtgtgaa cgagtgcgga gacaacagcg acgagcaggg ttgcatttgt 1400
ccggacccag accttcaggt gttccaatgg gaagtgcctc tcgaaaagcc 1450
agcagtgcaa tgggaaggac gactgtgggg acgggtccga cgaggcctcc 1500
tgccccaagg tgaacgtcgt cacttgtacc aaacacacct accgctgcct 1550
caatgggctc tgcttgagca agggcaaccc tgagtgtgac gggaaggagg 1600
actgtagcga cggctcagat gagaaggact gcgactgtgg gctgcggtca 1650
ttcacgagac aggctcgtgt tgttgggggc acggatgcgg atgagggcga 1700
gtggccctgg caggtaagcc tgcatgctct gggccagggc cacatctgcg 1750
gtgcttccct catctctccc aactggctgg tctctgccgc acactgctac 1800
atcgatgaca gaggattcag gtactcagac cccacgcagg acggccttcc 1850
tgggcttgca cgaccagagc cagcgcaggc cctggggtgc aggagcgcag 1900
gctcaagcgc atcatctccc accccttctt caatgacttc accttcgact 1950
atgacatcgc gctgctggag ctggagaaac cggcagagta cagctccatg 2000
gtgcggccca tctgcctgcc ggacgcctgc catgtcttcc ctgccggcaa 2050
ggccatctgg gtcacgggct ggggacacac ccagtatgga ggcactggcg 2100
cgctgatcct gcaaaagggt gagatccgcg tcatcaacca gaccacctgc 2150
gagaacctcc tgccgcagca gatcacgccg cgcatgatgt gcgtgggctt 2200
cctcagcggc ggcgtggact cctgccaggg tgattccggg ggacccctgt 2250
ccagcgtgga ggcggatggg cggatcttcc aggccggtgt ggtgagctgg 2300

ggagacgctg cgctcagagg aacaagccag gcgtgtacac aaggctccct 2350
ctgtttcggg aatggatcaa agagaacact ggggtatagg ggccggggcc 2400
acccaaatgt gtacacctgc ggggccaccc atcgtccacc ccagtgtgca 2450
cgcctgcagg ctggagactc gcgcaccgtg acctgcacca gcgccccaga 2500
acatacactg tgaactcatc tccaggctca aatctgctag aaaacctctc 2550
gcttcctcag cctccaaagt ggagctggga gggtagaagg ggaggaacac 2600
tggtggttct actgacccaa ctggggcaag gtttgaagca cagctccggc 2650
agcccaagtg ggcgaggacg cgtttgtgca tactgccctg ctctatacac 2700
ggaagacctg gatctctagt gagtgtgact gccggatctg gctgtggtcc 2750
ttggccacgc ttcttgagga agcccaggct cggaggaccc tggaaaacag 2800
acgggtctga gactgaaaat ggtttaccag ctcccaggtg acttcagtgt 2850
gtgtattgtg taaatgagta aaacatttta tttcttttta aaaaaaaaaa 2900

<210> 10
<211> 902
<212> PRT
<213> Mus musculus

<220>
<223> Epithin

<400> 10
Met Gly Ser Asn Arg Gly Arg Lys Ala Gly Gly Gly Ser Gln Asp
                5                   10                  15
Phe Gly Ala Gly Leu Lys Tyr Asp Ser Arg Leu Glu Asn Met Asn
                20                  25                  30
Gly Phe Glu Glu Gly Val Glu Phe Leu Pro Ala Asn Asn Ala Lys
                35                  40                  45
Lys Val Glu Lys Arg Gly Pro Arg Arg Trp Val Val Leu Val Ala
                50                  55                  60
Val Leu Phe Ser Phe Leu Leu Leu Ser Leu Met Ala Gly Leu Leu
                65                  70                  75
Val Trp His Phe His Tyr Arg Asn Val Arg Val Gln Lys Val Phe
                80                  85                  90
Asn Gly His Leu Arg Ile Thr Asn Glu Ile Phe Leu Asp Ala Tyr
                95                  100                 105

Glu Asn Ser Thr Ser Thr Glu Phe Ile Ser Leu Ala Ser Gln Val
                110                 115                 120
Lys Glu Ala Leu Lys Leu Leu Tyr Asn Glu Val Pro Val Leu Gly
                125                 130                 135
Pro Tyr His Lys Lys Ser Ala Val Thr Ala Phe Ser Glu Gly Ser
                140                 145                 150
Val Ile Ala Tyr Tyr Trp Ser Glu Phe Ser Ile Pro Pro His Leu
                155                 160                 165
Ala Glu Glu Val Asp Arg Ala Met Ala Val Glu Arg Val Val Thr
                170                 175                 180
Leu Pro Pro Arg Ala Arg Ala Leu Lys Ser Phe Val Leu Thr Ser
                185                 190                 195
Val Val Ala Phe Pro Ile Asp Pro Arg Met Leu Gln Arg Thr Gln
                200                 205                 210
Asp Asn Ser Cys Ser Phe Ala Leu His Ala His Gly Ala Ala Val
                215                 220                 225
Thr Arg Phe Thr Thr Pro Gly Phe Pro Asn Ser Pro Tyr Pro Ala
                230                 235                 240
His Ala Arg Cys Gln Trp Val Leu Arg Gly Asp Ala Asp Ser Val
                245                 250                 255
Leu Ser Leu Thr Phe Arg Ser Phe Asp Val Ala Pro Cys Asp Glu
                260                 265                 270
His Gly Ser Asp Leu Val Thr Val Tyr Asp Ser Leu Ser Pro Met
                275                 280                 285
Glu Pro His Ala Val Val Arg Leu Cys Gly Thr Phe Ser Pro Ser
                290                 295                 300
Tyr Asn Leu Thr Phe Leu Ser Ser Gln Asn Val Phe Leu Val Thr
                305                 310                 315
Leu Ile Thr Asn Thr Gly Arg Arg His Leu Gly Phe Glu Ala Thr
                320                 325                 330
Phe Phe Gln Leu Pro Lys Met Ser Ser Cys Gly Gly Val Leu Ser
                335                 340                 345
Asp Thr Gln Gly Thr Phe Ser Ser Pro Tyr Tyr Pro Gly His Tyr
                350                 355                 360
Pro Pro Asn Ile Asn Cys Thr Trp Asn Ile Lys Val Pro Asn Asn
                365                 370                 375
Arg Asn Val Lys Val Arg Phe Lys Leu Phe Tyr Leu Val Asp Pro
                380                 385                 390

Asn Val Pro Val Gly Ser Cys Thr Lys Asp Tyr Val Glu Ile Asn
                395                 400                 405
Gly Glu Lys Gly Ser Gly Glu Arg Ser Gln Phe Val Val Ser Ser
                410                 415                 420
Asn Ser Ser Lys Ile Thr Val His Phe His Ser Asp His Ser Tyr
                425                 430                 435
Thr Asp Thr Gly Phe Leu Ala Glu Tyr Leu Ser Tyr Asp Ser Asn
                440                 445                 450
Asp Pro Cys Pro Gly Met Phe Met Cys Lys Thr Gly Arg Cys Ile
                455                 460                 465
Arg Lys Glu Leu Arg Cys Asp Gly Trp Ala Asp Cys Pro Asp Tyr
                470                 475                 480
Ser Asp Glu Arg Tyr Cys Arg Cys Asn Ala Thr His Gln Phe Thr
                485                 490                 495
Cys Lys Asn Gln Phe Cys Lys Pro Leu Phe Trp Val Cys Asp Ser
                500                 505                 510
Val Asn Asp Cys Gly Asp Gly Ser Asp Glu Glu Gly Cys Ser Cys
                515                 520                 525
Pro Ala Gly Ser Phe Lys Cys Ser Asn Gly Lys Cys Leu Pro Gln
                530                 535                 540
Ser Gln Lys Cys Asn Gly Lys Asp Asn Cys Gly Asp Gly Ser Asp
                545                 550                 555
Glu Ala Ser Cys Asp Ser Val Asn Val Val Ser Cys Thr Lys Tyr
                560                 565                 570
Thr Tyr Arg Cys Gln Asn Gly Leu Cys Leu Ser Lys Gly Asn Pro
                575                 580                 585
Glu Cys Asp Gly Lys Thr Asp Cys Ser Asp Gly Ser Asp Glu Lys
                590                 595                 600
Asn Cys Asp Cys Gly Leu Arg Ser Phe Thr Lys Gln Ala Arg Val
                605                 610                 615
Val Gly Gly Thr Asn Ala Asp Glu Gly Glu Trp Pro Trp Gln Val
                620                 625                 630
Ser Leu His Ala Leu Gly Gln Gly His Leu Cys Gly Ala Ser Leu
                635                 640                 645
Ile Ser Pro Asp Trp Leu Val Ser Ala Ala His Cys Phe Gln Asp
                650                 655                 660
Asp Lys Asn Phe Lys Tyr Ser Asp Tyr Thr Met Trp Thr Ala Phe
                665                 670                 675

Leu Gly Leu Leu Asp Gln Ser Lys Arg Ser Ala Ser Gly Val Gln
                680                 685                 690
Glu Leu Lys Leu Lys Arg Ile Ile Thr His Pro Ser Phe Asn Asp
                695                 700                 705
Phe Thr Phe Asp Tyr Asp Ile Ala Leu Leu Glu Leu Glu Lys Ser
                710                 715                 720
Val Glu Tyr Ser Thr Val Val Arg Pro Ile Cys Leu Pro Asp Ala
                725                 730                 735
Thr His Val Phe Pro Ala Gly Lys Ala Ile Trp Val Thr Gly Trp
                740                 745                 750
Gly His Thr Lys Glu Gly Gly Thr Gly Ala Leu Ile Leu Gln Lys
                755                 760                 765
Gly Glu Ile Arg Val Ile Asn Gln Thr Thr Cys Glu Asp Leu Met
                770                 775                 780
Pro Gln Gln Ile Thr Pro Arg Met Met Cys Val Gly Phe Leu Ser
                785                 790                 795
Gly Gly Val Asp Ser Cys Gln Gly Asp Ser Gly Gly Pro Leu Ser
                800                 805                 810
Ser Ala Glu Lys Asp Gly Arg Met Phe Gln Ala Gly Val Val Ser
                815                 820                 825
Trp Gly Glu Gly Cys Ala Gln Arg Asn Lys Pro Gly Val Tyr Thr
                830                 835                 840
Arg Leu Pro Cys Ser Ser Gly Leu Asp Gln Arg Ala His Trp Gly
                845                 850                 855
Ile Ala Ala Trp Thr Asp Ser Arg Pro Gln Thr Pro Thr Gly Met
                860                 865                 870
Pro Asp Met His Thr Trp Ile Gln Glu Arg Asn Thr Asp Asp Ile
                875                 880                 885
Tyr Ala Val Ala Ser Pro Pro Gln His Asn Pro Asp Cys Glu Leu
                890                 895                 900
His Pro

<210>  11
<211>  23
<212>  DNA
<213>  Artificial sequence

<220>
<221> source
<223> /note= "Description of Artificial sequence: Degenerate
            oligonucleotide primer"

<220>
<221>  misc_feature
<222>  6, 9, 12, 15, 18
<223>  n=Inosine

<400>  11
tgggtngtna cngcngcnca ytg                                              23

<210>  12
<211>  20
<212>  DNA
<213>  Artificial sequence

<220>
<221> source
<223> /note= "Description of Artificial sequence: Degenerate
            oligonucleotide primer"

<220>
<221>  misc_feature
<222>  3, 6, 9, 12, 18
<223>  n=Inosine

<400>  12
arnggnccnc cnswrtcncc                                                  20

<210>  13
<211>  12
<212>  PRT
<213>  Homo sapiens

<220>
<223>  Fragment of TADG-15

<400>  13
Leu Phe Arg Asp Trp Ile Lys Glu Asn Thr Gly Val
                5                   10

<210>  14
<211>  20
<212>  DNA
<213>  Artificial sequence

<220>
<221> source
<223> /note= "Description of Artificial sequence: "TADG-15 forward
            oligonucleotide primer"

<400>  14
atgacagagg attcaggtac                                          20

<210>  15
<211>  20
<212>  DNA
<213>  Artificial sequence

<220>
<221> source
<223> /note= "Description of Artificial sequence: "TADG-15 reverse
            oligonucleotide primer"

<400>  15
gaaggtgaag tcattgaaga                                                    20

<210>  16
<211>  20
<212>  DNA
<213>  Artificial sequence

<220>
<221> source
<223> /note= "Description of Artificial sequence:  -tubulin forward
              oligonucleotide primer"

<400>  16
cgcatcaacg tgtactacaa                                                    20

<210>  17
<211>  20
<212>  DNA
<213>  Artificial sequence

<220>
<221> source
<223> /note= "Description of Artificial sequence:   -tubulin reverse
              oligonucleotide primer"

<400>  17
tacgagctgg tggactgaga                                                    20

<210>  18

<211>  3147

<212>  RNA

<213>  Artificial sequence

<220>

<221> source

<223> /note= "Description of Artificial sequence: Antisense of TADG-15"

<400>  18

uuuuuuuuuu uuuuuuuuua aaaagaaaua aauuguuuua cccauuuaca    50

caaauacaca cacugaaguc cacccuggga gcugguaaaa caauuucagu   100

cucagacccg ucuguuuucc aggguccucc gagccugggc uuccucaaga   150

gcguggccca agggccccac agcccagauc cggcagcccc accaccuuca   200

cugaggaggc uccgaagcuc cguucccgcu gcuccuuaca gacaggggag   250

gcagauauac acaaacgcgc cucggcccag cuuggggcug gcgggggagg   300

cugugucuuc aaaccuuugc ccccaguugg gucaguagaa ccaccagugu   350

ccuccccuuc uaccucccag cuccacuuug gaggcugagg aagcgagagg   400

uuuucuaggc agauuuggag cccuggagau ugaguucaca guguauguuc   450

ugggggcgcu ggugcaguca gcgguccagu cuccagccug caggcgugca   500

cacuggggug gacgaugggu ggccccgcag guguacacau uugguggccc   550

ccggccccua uaccccagug uucucuuuga uccagucccg aaacagaggg   600

agccuugugu acacgccugg cuuguuccuc ugagcgcagc cgucucccca   650

gcucaccaca ccggccugga agauccgccc auccgccucc acgcuggaca   700

gggguccccc ggaaucaccc uggcaggagu ccacgccgcc gcugaggaag   750

cccacgcaca ucaugcgcgg cgugaucugc ugcggcagga gguucucgca   800

gguggucugg uugaugacgc ggaucucacc cuuuugcagg aucagcgcgc   850

cagugccucc auacugggug uguccccagc ccgugaccca gauggccuug   900

ccggcaggga agacauggga ggcguccggc aggcagaugg gccgcaccau   950

ggagcuguac ucugccgguu ucuccagcuc cagcagcgcg augucauagu  1000

cgaaggugaa gucauugaag aagggguggg agaugaugcg cuugagccug  1050

cgcuccugca ccccaggggc gcugcgcugg cucuggucgu gcaagcccag  1100

gaaggccguc cacugcgugg ggucugagua ccugaauccu cugucaucga  1150

uguagcagug ugcggcagag accagccagu ugggagagau gagggaagca  1200

ccgcagaugu ggcccuggcc cagagcaugc aggcuuaccu gccagggcca  1250

cucgcccuca uccgcauccg ugcccccaac aacacgagcc ugucucguga  1300

augaccgcag cccacagucg caguccuucu caucugagcc gucgcuacag  1350

uccuccuucc cgucacacuc aggguugccc uugcucaagc agagcccauu 1400
gaggcagcgg uagguguguu ugguacaagu gacgacguuc accuuggggc 1450
aggaggccuc gucggacccg uccccacagu cguccuuccc auugcacugc 1500
uggcuuuucg agaggcacuu cccauuggaa caccugaagg ucugggccgg 1550
acaacugcac cccugcucgu cgcuguuguc uccgcagucg uucacacugu 1600
cgcagaccca gaagaggggc uugcagaacu uguucuugca cgugaacugg 1650
uggccggcgu cgcaacugca guugagcuca ucgcuguggu cggugcaguc 1700
ggcccagcca ucacagcgca gcuccuuccg gauacaccgc cccgugcggc 1750
acgugaacug ccccgggcau gggucacugg agucguagga gagguauuca 1800
gcuaagaagc cggugucggu guaggacuga ucugagugga agcgaacugu 1850
gaucuuguug cuguugcugg ugacgacgaa cugggaccuc ucuccgcagu 1900
auuucucccc auugaucucc acguaguccu uggggcaggu gcccgcaggc 1950
acgccgggcu ccagcaggua gaagaauuug aagcucaccu ucacaugcug 2000
guuguugggc accucaaugu uccaugugca gucaauguug gguggguagu 2050
ggccugggua guaggggcug uugaaugucc ccugggcuuu acguaagcgg 2100
ccuccacagc ugcucauccu aggcagcugg aagaaggugg ccucaaagcc 2150
gggaugccgc cgcucagugu ugguuaucag ugugaugagc aggacguucu 2200
gggaggagug gaaggucagg uuguaggagg gaggguaggu gccacacaac 2250
ugcaccaggg cguggggcuc cauggggcuc aggguguugu acaccgucac 2300
caggucgcug ccgcgcucgu cgcaggacgc aaggucaaag cugcggaagg 2350
ugaggcucag cacugagucg gcgucccccc gcagggccca cuggcagcgg 2400
gcaugagcgg gguaggggcu gucagggaag ccgggcgugg ugaagcgcau 2450
cagcuccaca ccgcgggcgu gcaggccaaa gcugcagcug uuguccuggg 2500
uccucuguac uguuuuggag uccgugggga aagccaccac ugaggugacc 2550
acaaaggacu ucagggagcg cgcccgcggg ggcagcauga cuacgcgcuc 2600
cucggccaug acgcgcucgg ccuccuccac caggugcugc gggaugcuga 2650
acucagacca guaguaggcg augacgcugc ccucgcugaa ggccgucaca 2700
gccgacuccu ugugguaggg gcccaggaau gggacuccgc uguacagcag 2750
cuucagcgcg uccuucaccu ugcuggccag gcuuacaaac ucaguggagu 2800
uggaguucuc guaggcaucc acaaaauucu cauuugugau ccucauguag 2850
ccauugaaga ccuucuggac acgcacgucc cgguacugca aaugccacac 2900
caggaagccg auccccagca agaccaagag gaggccgauc agcacggcug 2950
ccagcaccac ccagcgcccc gggccaugcu uuuccaccuu cuugacguug 3000
uugacuggca ggaacuccac gccuuccucc aagccauuca cuuucucgug 3050
ccgggaguug uacuugaguc ccgcgccgaa guccuucggg cccccuccgc 3100
ccuugcgggc ccgaucgcuc cccaugguac cccgaggccg cucuuga 3147

<210> 19
<211> 9
<212> PRT
<213> Homo sapiens

<220>
<223> Residues 68-76 of the TADG-15 protein

<400> 19
Val Leu Leu Gly Ile Gly Phe Leu Val
                5

<210> 20
<211> 9
<212> PRT
<213> Homo sapiens

<220>
<223> Residues 126-134 of the TADG-15 protein

<400> 20
Leu Leu Tyr Ser Gly Val Pro Phe Leu
                5

<210> 21
<211> 9
<212> PRT
<213> Homo sapiens

<220>
<223> Residues 644-652 of the TADG-15 protein

<400> 21
Ser Leu Ile Ser Pro Asn Trp Leu Val
                5

<210>  22

<211>  9

<212>  PRT

<213>  Homo sapiens

<220>

<223>  Residues 379-387 of the TADG-15 protein

<400>  22

Lys Val Ser Phe Lys Phe Phe Tyr Leu

                    5

<210>  23

<211>  9

<212>  PRT

<213>  Homo sapiens

<220>

<223>  Residues 386-394 of the TADG-15 protein

<400>  23

Tyr Leu Leu Glu Pro Gly Val Pro Ala

                    5

<210>  24

<211>  9

<212>  PRT

<213>  Homo sapiens

<220>

<223>  Residues 257-265 of the TADG-15 protein

<400>  24

Ser Leu Thr Phe Arg Ser Phe Asp Leu

                    5

<210> 25
<211> 9
<212> PRT
<213> Homo sapiens

<220>
<223> Residues 762-770 of the TADG-15 protein

<400> 25
Ile Leu Gln Lys Gly Glu Ile Arg Val
                5

<210> 26
<211> 9
<212> PRT
<213> Homo sapiens

<220>
<223> Residues 841-849 of the TADG-15 protein

<400> 26
Arg Leu Pro Leu Phe Arg Asp Trp Ile
                5

<210> 27
<211> 9
<212> PRT
<213> Homo sapiens

<220>
<223> Residues 64-72 of the TADG-15 protein

<400> 27
Gly Leu Leu Leu Val Leu Leu Gly Ile
                5

<210> 28
<211> 9
<212> PRT
<213> Homo sapiens

<220>
<223> Residues 57-65 of the TADG-15 protein

<400> 28
Val Leu Ala Ala Val Leu Ile Gly Leu
                5

<210> 29
<211> 9
<212> PRT
<213> Homo sapiens

<220>
<223> Residues 67-75 of the TADG-15 protein

<400> 29
Leu Val Leu Leu Gly Ile Gly Phe Leu
                5

<210> 30
<211> 9
<212> PRT
<213> Homo sapiens

<220>
<223> Residues 379-387 of the TADG-15 protein

<400> 30
Lys Val Ser Phe Lys Phe Phe Tyr Leu
                5

<210>  31
<211>  9
<212>  PRT
<213>  Homo sapiens

<220>
<223>  Residues 126-134 of the TADG-15 protein

<400>  31
Leu Leu Tyr Ser Gly Val Pro Phe Leu
                5

<210>  32
<211>  9
<212>  PRT
<213>  Homo sapiens

<220>
<223>  Residues 88-96 of the TADG-15 protein

<400>  32
Lys Val Phe Asn Gly Tyr Met Arg Ile
                5

<210>  33
<211>  9
<212>  PRT
<213>  Homo sapiens

<220>
<223>  Residues 670-678 of the TADG-15 protein

<400>  33
Thr Gln Trp Thr Ala Phe Leu Gly Leu
                5

<210> 34
<211> 9
<212> PRT
<213> Homo sapiens

<220>
<223> Residues 119-127 of the TADG-15 protein

<400> 34
Lys Val Lys Asp Ala Leu Lys Leu Leu
                5

<210> 35
<211> 9
<212> PRT
<213> Homo sapiens

<220>
<223> Residues 60-68 of the TADG-15 protein

<400> 35
Ala Val Leu Ile Gly Leu Leu Leu Val
                5

<210> 36
<211> 9
<212> PRT
<213> Homo sapiens

<220>
<223> Residues 62-70 of the TADG-15 protein

<400> 36
Leu Ile Gly Leu Leu Leu Val Leu Leu
                5

<210>  37
<211>  9
<212>  PRT
<213>  Homo sapiens

<220>
<223>  Residues 57-65 of the TADG-15 protein

<400>  37
Val Leu Ala Ala Val Leu Ile Gly Leu
                    5

<210>  38
<211>  9
<212>  PRT
<213>  Homo sapiens

<220>
<223>  Residues 61-69 of the TADG-15 protein

<400>  38
Val Leu Ile Gly Leu Leu Leu Val Leu
                    5

<210>  39
<211>  9
<212>  PRT
<213>  Homo sapiens

<220>
<223>  Residues 146-154 of the TADG-15 protein

<400>  39
Phe Ser Glu Gly Ser Val Ile Ala Tyr
                    5

<210> 40
<211> 9
<212> PRT
<213> Homo sapiens

<220>
<223> Residues 658-666 of the TADG-15 protein

<400> 40
Tyr Ile Asp Asp Arg Gly Phe Arg Tyr
                5

<210> 41
<211> 9
<212> PRT
<213> Homo sapiens

<220>
<223> Residues 449-457 of the TADG-15 protein

<400> 41
Ser Ser Asp Pro Cys Pro Gly Gln Phe
                5

<210> 42
<211> 9
<212> PRT
<213> Homo sapiens

<220>
<223> Residues 401-409 of the TADG-15 protein

<400> 42
Tyr Val Glu Ile Asn Gly Glu Lys Tyr
                5

<210>  43
<211>  9
<212>  PRT
<213>  Homo sapiens

<220>
<223>  Residues 387-395 of the TADG-15 protein

<400>  43
Leu Leu Glu Pro Gly Val Pro Ala Gly
                5

<210>  44
<211>  9
<212>  PRT
<213>  Homo sapiens

<220>
<223>  Residues 553-561 of the TADG-15 protein

<400>  44
Gly Ser Asp Glu Ala Ser Cys Pro Lys
                5
<210>  45
<211>  9
<212>  PRT
<213>  Homo sapiens

<220>
<223>  Residues 97-105 of the TADG-15 protein

<400>  45
Thr Asn Glu Asn Phe Val Asp Ala Tyr
                5

<210>  46
<211>  9
<212>  PRT
<213>  Homo sapiens

<220>
<223>  Residues 110-118 of the TADG-15 protein

<400>  46
Ser Thr Glu Phe Val Ser Leu Ala Ser
                5

<210>  47
<211>  9
<212>  PRT
<213>  Homo sapiens

<220>
<223>  Residues 811-819 of the TADG-15 protein

<400>  47
Ser Val Glu Ala Asp Gly Arg Ile Phe
                5

<210>  48
<211>  9
<212>  PRT
<213>  Homo sapiens

<220>
<223>  Residues 666-674 of the TADG-15 protein

<400>  48
Tyr Ser Asp Pro Thr Gln Trp Thr Ala
                5

<210> 49
<211> 9
<212> PRT
<213> Homo sapiens

<220>
<223> Residues 709-717 of the TADG-15 protein

<400> 49
Asp Tyr Asp Ile Ala Leu Leu Glu Leu
                5

<210> 50
<211> 9
<212> PRT
<213> Homo sapiens

<220>
<223> Residues 408-416 of the TADG-15 protein

<400> 50
Lys Tyr Cys Gly Glu Arg Ser Gln Phe
                5

<210> 51
<211> 9
<212> PRT
<213> Homo sapiens

<220>
<223> Residues 754-762 of the TADG-15 protein

<400> 51
Gln Tyr Gly Gly Thr Gly Ala Leu Ile
                5

<210>  52
<211>  9
<212>  PRT
<213>  Homo sapiens

<220>
<223>  Residues 153-161 of the TADG-15 protein

<400>  52
Ala Tyr Tyr Trp Ser Glu Phe Ser Ile
                5

<210>  53
<211>  9
<212>  PRT
<213>  Homo sapiens

<220>
<223>  Residues 722-730 of the TADG-15 protein

<400>  53
Glu Tyr Ser Ser Met Val Arg Pro Ile
                5

<210>  54
<211>  9
<212>  PRT
<213>  Homo sapiens

<220>
<223>  Residues 326-334 of the TADG-15 protein

<400>  54
Gly Phe Glu Ala Thr Phe Phe Gln Leu
                5

<210>  55
<211>  9
<212>  PRT
<213>  Homo sapiens

<220>
<223>  Residues 304-312 of the TADG-15 protein

<400>  55
Thr Phe His Ser Ser Gln Asn Val Leu
                5

<210>  56
<211>  9
<212>  PRT
<213>  Homo sapiens

<220>
<223>  Residues 707-715 of the TADG-15 protein

<400>  56
Thr Phe Asp Tyr Asp Ile Ala Leu Leu
                5

<210>  57
<211>  9
<212>  PRT
<213>  Homo sapiens

<220>
<223>  Residues 21-29 of the TADG-15 protein

<400>  57
Lys Tyr Asn Ser Arg His Glu Lys Val
                5

<210>  58
<211>  9
<212>  PRT
<213>  Homo sapiens

<220>
<223>  Residues 665-673 of the TADG-15 protein

<400>  58
Arg Tyr Ser Asp Pro Thr Gln Trp Thr
                5

<210>  59
<211>  9
<212>  PRT
<213>  Homo sapiens

<220>
<223>  Residues 686-694 of the TADG-15 protein

<400>  59
Ala Pro Gly Val Gln Glu Arg Arg Leu
                5

<210>  60
<211>  9
<212>  PRT
<213>  Homo sapiens

<220>
<223>  Residues 12-20 of the TADG-15 protein

<400>  60
Gly Pro Lys Asp Phe Gly Ala Gly Leu
                5

<210>  61
<211>  9
<212>  PRT
<213>  Homo sapiens

<220>
<223>  Residues 668-676 of the TADG-15 protein

<400>  61
Asp Pro Thr Gln Trp Thr Ala Phe Leu
                5

<210>  62
<211>  9
<212>  PRT
<213>  Homo sapiens

<220>
<223>  Residues 461-469 of the TADG-15 protein

<400>  62
Thr Gly Arg Cys Ile Arg Lys Glu Leu
                5

<210>  63
<211>  9
<212>  PRT
<213>  Homo sapiens

<220>
<223>  Residues 59-67 of the TADG-15 protein

<400>  63
Ala Ala Val Leu Ile Gly Leu Leu Leu
                5

<210>  64
<211>  9
<212>  PRT
<213>  Homo sapiens

<220>
<223>  Residues 379-387 of the TADG-15 protein

<400>  64
Lys Val Ser Phe Lys Phe Phe Tyr Leu
                5

<210>  65
<211>  9
<212>  PRT
<213>  Homo sapiens

<220>
<223>  Residues 119-127 of the TADG-15 protein

<400>  65
Lys Val Lys Asp Ala Leu Lys Leu Leu
                5

<210>  66
<211>  9
<212>  PRT
<213>  Homo sapiens

<220>
<223>  Residues 780-788 of the TADG-15 protein

<400>  66
Leu Pro Gln Gln Ile Thr Pro Arg Met
                5

<210>  67
<211>  9
<212>  PRT
<213>  Homo sapiens

<220>
<223>  Residues 67-75 of the TADG-15 protein

<400>  67
Leu Val Leu Leu Gly Ile Gly Phe Leu
                5

<210>  68
<211>  9
<212>  PRT
<213>  Homo sapiens

<220>
<223>  Residues 283-291 of the TADG-15 protein

<400>  68
Ser Pro Met Glu Pro His Ala Leu Val
                5

<210>  69
<211>  9
<212>  PRT
<213>  Homo sapiens

<220>
<223>  Residues 12-20 of the TADG-15 protein

<400>  69
Gly Pro Lys Asp Phe Gly Ala Gly Leu
                5

<210>  70
<211>  9
<212>  PRT
<213>  Homo sapiens

<220>
<223>  Residues 257-265 of the TADG-15 protein

<400>  70
Ser Leu Thr Phe Arg Ser Phe Asp Leu
                5

<210>  71
<211>  9
<212>  PRT
<213>  Homo sapiens

<220>
<223>  Residues 180-188 of the TADG-15 protein

<400>  71
Met Leu Pro Pro Arg Ala Arg Ser Leu
                5

<210>  72
<211>  9
<212>  PRT
<213>  Homo sapiens

<220>
<223>  Residues 217-225 of the TADG-15 protein

<400>  72
Gly Leu His Ala Arg Gly Val Glu Leu
                5

<210> 73
<211> 9
<212> PRT
<213> Homo sapiens

<220>
<223> Residues 173-181 of the TADG-15 protein

<400> 73
Met Ala Glu Glu Arg Val Val Met Leu
                5

<210> 74
<211> 9
<212> PRT
<213> Homo sapiens

<220>
<223> Residues 267-275 of the TADG-15 protein

<400> 74
Ser Cys Asp Glu Arg Gly Ser Asp Leu
                5

<210> 75
<211> 9
<212> PRT
<213> Homo sapiens

<220>
<223> Residues 567-575 of the TADG-15 protein

<400> 75
Cys Thr Lys His Thr Tyr Arg Cys Leu
                5

<210>  76
<211>  9
<212>  PRT
<213>  Homo sapiens

<220>
<223>  Residues 724-732 of the TADG-15 protein

<400>  76
Ser Ser Met Val Arg Pro Ile Cys Leu
                5

<210>  77
<211>  9
<212>  PRT
<213>  Homo sapiens

<220>
<223>  Residues 409-417 of the TADG-15 protein

<400>  77
Tyr Cys Gly Glu Arg Ser Gln Phe Val
                5

<210>  78
<211>  9
<212>  PRT
<213>  Homo sapiens

<220>
<223>  Residues 495-503 of the TADG-15 protein

<400>  78
Thr Cys Lys Asn Lys Phe Cys Lys Pro
                5

<210>  79
<211>  9
<212>  PRT
<213>  Homo sapiens

<220>
<223>  Residues 427-435 of the TADG-15 protein

<400>  79
Val Arg Phe His Ser Asp Gln Ser Tyr
                5

<210>  80
<211>  9
<212>  PRT
<213>  Homo sapiens

<220>
<223>  Residues 695-703 of the TADG-15 protein

<400>  80
Lys Arg Ile Ile Ser His Pro Phe Phe
                5

<210>  81
<211>  9
<212>  PRT
<213>  Homo sapiens

<220>
<223>  Residues 664-672 of the TADG-15 protein

<400>  81
Phe Arg Tyr Ser Asp Pro Thr Gln Trp
                5

<210>  82
<211>  9
<212>  PRT
<213>  Homo sapiens

<220>
<223>  Residues 220-228 of the TADG-15 protein

<400>  82
Ala Arg Gly Val Glu Leu Met Arg Phe
                5

<210>  83
<211>  9
<212>  PRT
<213>  Homo sapiens

<220>
<223>  Residues 492-500 of the TADG-15 protein

<400>  83
His Gln Phe Thr Cys Lys Asn Lys Phe
                5

<210>  84
<211>  9
<212>  PRT
<213>  Homo sapiens

<220>
<223>  Residues 53-61 of the TADG-15 protein

<400>  84
Gly Arg Trp Val Val Leu Ala Ala Val
                5

<210>  85
<211>  9
<212>  PRT
<213>  Homo sapiens

<220>
<223>  Residues 248-256 of the TADG-15 protein

<400>  85
Leu Arg Gly Asp Ala Asp Ser Val Leu
                5

<210>  86
<211>  9
<212>  PRT
<213>  Homo sapiens

<220>
<223>  Residues 572-580 of the TADG-15 protein

<400>  86
Tyr Arg Cys Leu Asn Gly Leu Cys Leu
                5

<210>  87
<211>  9
<212>  PRT
<213>  Homo sapiens

<220>
<223>  Residues 692-700 of the TADG-15 protein

<400>  87
Arg Arg Leu Lys Arg Ile Ile Ser His
                5

<210>  88
<211>  9
<212>  PRT
<213>  Homo sapiens

<220>
<223>  Residues 24-32 of the TADG-15 protein

<400>  88
Ser Arg His Glu Lys Val Asn Gly Leu
                5

<210>  89
<211>  9
<212>  PRT
<213>  Homo sapiens

<220>
<223>  Residues 147-155 of the TADG-15 protein

<400>  89
Ser Glu Gly Ser Val Ile Ala Tyr Tyr
                5

<210>  90
<211>  9
<212>  PRT
<213>  Homo sapiens

<220>
<223>  Residues 715-723 of the TADG-15 protein

<400>  90
Leu Glu Leu Glu Lys Pro Ala Glu Tyr
                5

<210>  91
<211>  9
<212>  PRT
<213>  Homo sapiens

<220>
<223>  Residues 105-113 of the TADG-15 protein

<400>  91
Tyr Glu Asn Ser Asn Ser Thr Glu Phe
                5

<210>  92
<211>  9
<212>  PRT
<213>  Homo sapiens

<220>
<223>  Residues 14-22 of the TADG-15 protein

<400>  92
Lys Asp Phe Gly Ala Gly Leu Lys Tyr
                5

<210>  93
<211>  9
<212>  PRT
<213>  Homo sapiens

<220>
<223>  Residues 129-137 of the TADG-15 protein

<400>  93
Ser Gly Val Pro Phe Leu Gly Pro Tyr
                5

<210> 94
<211> 9
<212> PRT
<213> Homo sapiens

<220>
<223> Residues 436-444 of the TADG-15 protein

<400> 94
Thr Asp Thr Gly Phe Leu Ala Glu Tyr
                5

<210> 95
<211> 9
<212> PRT
<213> Homo sapiens

<220>
<223> Residues 766-774 of the TADG-15 protein

<400> 95
Gly Glu Ile Arg Val Ile Asn Gln Thr
                5

<210> 96
<211> 9
<212> PRT
<213> Homo sapiens

<220>
<223> Residues 402-410 of the TADG-15 protein

<400> 96
Val Glu Ile Asn Gly Glu Lys Tyr Cys
                5

<210> 97

<211> 9

<212> PRT

<213> Homo sapiens

<220>

<223> Residues 482-490 of the TADG-15 protein

<400> 97

Asp Glu Leu Asn Cys Ser Cys Asp Ala

                5

<210> 98

<211> 9

<212> PRT

<213> Homo sapiens

<220>

<223> Residues 82-90 of the TADG-15 protein

<400> 98

Arg Asp Val Arg Val Gln Lys Val Phe

                5